# EUROPEAN PATENT APPLICATION

(11) **EP 3 567 112 A1**
(43) Date of publication of application: **13.11.2019**
(21) Application number: 19160013.9
(22) Date of filing: 12.06.2014
(51) Int. Cl.: C12N 15/86, A61K 48/00

(54) **MESSENGER RNA BASED VIRAL PRODUCTION**

(30) Priority: 13.06.2013 US 201361834512 P
(62) Divisional of application: 14736225.5
(71) Applicant: Translate Bio, Inc., Lexington, MA 02421 (US)
(72) Inventor: HEARTLEIN, Michael, Boxborough, MA 01719 (US); DEROSA, Frank, Lexington, MA 02421 (US); SMITH, Lianne, Lexington, MA 02421 (US)
(74) Representative: Goodfellow, Hugh Robin

(57) **Abstract**

The present invention provides methods for producing recombinant viral particles based on the use of exogenous mRNAs to supply various helper factors for assembly of viral particles, purified recombinant viral particles produced using such methods, and methods of using such viral particles.

SEQUENCE LISTING

<110>   TRANSLATE BIO, INC.

<120>   MESSENGER RNA BASED VIRAL PRODUCTION

<130>   P074857EP

<140>   Not yet assigned
<141>   2014-06-12

<150>   61/834,512
<151>   2013-06-13

<150>   14736225.5
<151>   2014-06-12

<160>   1

<170>   SeqWin2010, version 1.0

<210>   1
<211>   124
<212>   DNA
<213>   Artificial Sequence

<220>
<223>   Synthetic polynucleotide

<400>   1
tttaaaagaa aaggggggat tggggggtac agtgcagggg aaagaatagt agacataata  60
gcaacagaca tacaaactaa agaattacaa aaacaaatta caaaaattca aaattttcgg  120
gttt                                                              124

## Description

### CROSS REFERENCE TO RELATED APPLICATIONS

This application claims the benefit of U.S. Provisional Application No. 61/834,512, filed June 13, 2013, the contents of which are hereby incorporated by reference herein in their entirety.

### BACKGROUND

Recombinant viruses are commonly used to produce viral vaccines or viral vectors in gene therapy to deliver therapeutic genes to cells for the treatment of diseases, disorders or conditions typically associated with genetic defects. Current manufacturing systems for viruses for use in gene therapy or vaccine development typically require the establishment of separate stocks of recombinant plasmids or helper viruses to provide the viral enzymes, structural proteins, and other factors required for the assembly of virus. The current system typically also require simultaneous transfection/transduction of packaging cells with multiple helper plasmids/viruses. This process can be complicated, labor intensive and overwhelming to the packaging cells. It also carries the risk for generating replication competent viruses caused by recombination among helper plasmids or co-packaging of helper DNA sequences into the resulting infectious viral particles.

### SUMMARY

The present invention provides a simplified, more efficient and safer process for generating recombinant viruses with improved potency. In particular, the present invention provides a process in which individual viral helper factors are supplied to packaging cells by exogenous mRNAs (e.g., *in vitro* transcribed mRNAs) encoding such factors directly. Thus, the present invention allows for the production of recombinant or engineered viruses, in particular, lentivirus or adeno-associated viruses, without the need for transfection or transduction of packaging cells with multiple individual plasmids or viruses encoding helper factors. Prior to the present invention, production of recombinant viruses typically involves transfection of plasmids or transduction of viruses into packaging cells. Once inside packaging cells, plasmid/virus DNAs are first transported to the nucleus where they are transcribed into mRNAs. mRNAs are then transported to the cytoplasm so that they can be translated into the proteins with helper functions encoded by the plasmid/virus DNAs. By contrast, exogenous mRNAs, once transfected into a packaging cell, are translated into helper factors directly upon entry into the cytoplasm, thus eliminating the need for transport to nucleus, transcription, processing, and transport to cytoplasm prior to translation into proteins. Therefore, mRNA-based approach according to the present invention provides a simplified and more efficient production process. The elimination of multiple processing steps by the use of mRNA may also improve the ratio of full to empty capsids/virions, thus improving potency of the resulting viruses. In addition, mRNA-based production according to the present invention also eliminates the risk for generating replication competent virions caused by recombination among helper plasmids and/or co-packaging of helper DNAs, significantly improving safety of virus-based vaccines and gene therapy. Thus, the present invention represents a significant advancement in the field of recombinant viral production.

In one aspect, the present invention provides a method of producing a lentiviral particle by introducing into a packaging cell one or more exogenous mRNAs encoding one or more helper factors for assembling transduction-competent lentiviral particles, wherein the packaging cell comprises a gene of interest; and culturing the packaging cell under conditions suitable for the packaging cell to produce a lentiviral particle comprising the gene of interest.

In some embodiments, the one or more exogenous mRNAs are *in vitro* transcribed mRNAs or synthetic mRNAs. In some embodiments, the exogenous mRNAs are stabilized mRNAs (e.g., mRNAs containing 5' cap and/or 3' poly(A) tail, modified nucleotides, sugars and/or phosphate groups). In some embodiments, the one or more exogenous mRNAs are introduced by electroporation, lipofection, PEI, or combination thereof.

In some embodiments, the one or more helper factors are selected from the group consisting of a Pol protein, a Gag protein, an Env protein, and a combination thereof. In some embodiments, the one or more helper factors comprise a Pol protein, a Gag protein, and an Env protein. In some embodiments, the one or more helper factors further comprise a lentiviral Tat protein and/or a lentiviral Rev protein. In some embodiments, the one or more helper factors are encoded by one single exogenous mRNA molecule. In some embodiments, the one or more helper factors are encoded by two or more exogenous mRNA molecules. In some embodiments, each of the one or more helper factors is encoded by a separate individual exogenous mRNA molecule.

In some embodiments, the gene of interest is integrated in the genome of the packaging cell. In some embodiments, the gene of interest is present on an extra-chromosomal construct such as a plasmid. In some embodiments, the gene of interest is transiently transfected into the packaging cell. In some embodiments, the gene of interest is associated with a packaging signal. In some embodiments, the gene of interest is operably linked to a promoter. In some embodiments, the gene of interest is framed by a left and a right long terminal repeat (LTR) sequence.

In some embodiments, a suitable packaging cell is a mammalian cell. In some embodiments, a suitable mammalian cell is a human cell. In some embodiments, a suitable human cell is a HT-1080 cell. In some embodiments, a suitable mammalian cell is a CHO cell.

In some embodiments, a method according to the present invention further comprises a step of purifying the lentiviral particles.

In particular embodiments, the present invention provides a method of producing a lentiviral particle by introducing into a packaging cell (i) an exogenous mRNA encoding a lentiviral Gag protein, (ii) an exogenous mRNA encoding a lentiviral Pol protein, and (iii) an exogenous mRNA encoding a lentiviral Env protein; wherein the packaging cell comprises a gene of interest associated with a packaging signal; and culturing the packaging cell under conditions suitable for the packaging cell to produce a lentiviral particle comprising the gene of interest.

In various embodiments, the present invention provides a population of lentiviral particles produced using methods described herein.

In various embodiments, the present invention also provides a packaging cell capable of producing a lentiviral particle, comprising one or more exogenous mRNAs (e.g., in vitro transcribed or synthetic mRNAs) encoding one or more helper factors for assembling transduction-competent lentiviral particles, wherein the packaging cell comprises a gene of interest associated with a packaging signal.

In some embodiments, the present invention provides a system for producing a lentiviral particle, comprising one or more constructs encoding one or more helper factors for assembling transduction-competent lentiviral particles; reagents for *in vitro* transcription of mRNAs from the one or more constructs encoding one or more helper factors; a packaging cell; and reagents for introducing the *in vitro* transcribed mRNAs into the packaging cell. In some embodiments, the packaging cell comprises a gene of interest (e.g., stably integrated in the genome or transiently present on an extra- chromosomal plasmid).

In another aspect, the present invention provides a method of producing an adeno-associated virus (AAV) particle by introducing into a packaging cell one or more exogenous mRNAs encoding one or more helper factors for assembling transduction-competent AAV particles, wherein the packaging cell comprises a gene of interest; and culturing the packaging cell under conditions suitable for the packaging cell to produce an AAV particle comprising the gene of interest.

In some embodiments, the one or more exogenous mRNAs are *in vitro* transcribed mRNAs or synthetic mRNAs. In some embodiments, the exogenous mRNAs are stabilized mRNAs (e.g., mRNAs containing 5' cap and/or 3' poly(A) tail, modified nucleotides, sugars and/or phosphate groups). In some embodiments, the one or more exogenous mRNAs are introduced by electroporation, lipofection, PEI, or combination thereof.

In some embodiments, the one or more helper factors are selected from the group consisting of a Rep78 protein, a Rep 52 protein, a capsid (e.g., VP1, VP2 or VP3) protein, AAP, and combinations thereof. In some embodiments, the one or more helper factors comprise a Rep78 protein, a Rep 52 protein, a capsid protein, and AAP. In some embodiments, the one or more helper factors are encoded by one single exogenous mRNA molecule. In some embodiments, the one or more helper factors are encoded by two or more exogenous mRNA molecules. In some embodiments, each of the one or more helper factors is encoded by a separate individual exogenous mRNA molecule. In some embodiments, the one or more exogenous mRNAs are introduced by electroporation, lipofection, PEI, or combination thereof.

In some embodiments, the gene of interest is integrated in the genome of the packaging cell. In some embodiments, the gene of interest is present on a extra-chromosomal construct such as a plasmid. In some embodiments, the gene of interest is transiently transfected into the packaging cell. In some embodiments, the gene of interest is associated with a packaging signal. In some embodiments, the gene of interest is operably linked to a promoter. In some embodiments, the gene of interest is framed by a left and a right inverted terminal repeat (ITR) sequence.

In some embodiments, the AAV particle is an AAV1, AAV2, AAV3, AAV4, AAV5, AAV6, AAV7, AAV8, AAV9, AAV10, or AAV11 particle.

In some embodiments, a suitable packaging cell is a mammalian cell. In some embodiments, a suitable mammalian cell is a human cell. In some embodiments, a suitable human cell is a HEK293 cell.

In some embodiments, a suitable packaging cell is an insect cell. In some embodiments, a suitable insect cell is an SF9 cell.

In some embodiments, a method according to the present invention further comprises a step of purifying the AAV particles.

In particular embodiments, the present invention provides a method of producing an AAV particle by introducing into a packaging cell (i) an exogenous mRNA encoding a Rep 78 protein, (ii) an exogenous mRNA encoding a Rep 52 protein, and (iii) an exogenous mRNA encoding a capsid (e.g., VP1, VP2 or VP3) protein; wherein the packaging cell comprises a gene of interest associated with a packaging signal; and culturing the packaging cell under conditions suitable for the packaging cell to produce an AAV particle comprising the gene of interest.

In various embodiments, the present invention provides a population of AAV particles produced using methods described herein.

In various embodiments, the present invention provides a packaging cell capable of producing an AAV particle, comprising one or more exogenous mRNAs (e.g., *in vitro* transcribed mRNAs or synthetic mRNAs) encoding one or more helper factors for assembling transduction-competent AAV particles, wherein the packaging cell comprises a gene of interest associated with a packaging signal.

In various embodiments, the present invention provides a system for producing an AAV particle, comprising one or more constructs encoding one or more helper factors for assembling transduction-competent AAV particles; reagents for *in vitro* transcription of mRNAs from the one or more constructs encoding one or more helper factors; a packaging cell; and reagents for introducing the *in vitro* transcribed mRNAs into the packaging cell. In some embodiments, the packaging cell comprises a gene of interest.

Among other things, the present invention also provides methods of treating diseases, disorders or conditions (e.g., lysosomal storage diseases) by administering to a subject in need of treatment an effective amount of a lentiviral particle or an AAV particle containing a therapeutic gene of interest (e.g., a lysosomal enzyme) produced by methods described herein.

It is contemplated that inventive methods, systems, cells and compositions described herein can be used to produce any recombinant viral particles, viral vectors, viral vaccines or other viral products, including but not limited to, various retroviruses, adeno-viruses, adeno-associated viruses based particles, vectors, vaccines or other products. Examples of various recombinant viruses are provided in the detailed description. In some embodiments, a recombinant virus is not moloney murine leukemia virus.

It is also contemplated that mRNA based approach described herein can be used in combination with helper plasmids or viruses. For example, certain helper factors may be supplied by exogenous mRNAs while other helper factors may be provided by helper plasmids or viruses.

Other features, objects, and advantages of the present invention are apparent in the detailed description that follows. It should be understood, however, that the detailed description, while indicating embodiments of the present invention, is given by way of illustration only, not limitation. Various changes and modifications within the scope of the invention will become apparent to those skilled in the art from the detailed description.

### BRIEF DESCRIPTION OF THE DRAWINGS

The present teachings described herein will be more fully understood from the following description of various illustrative embodiments, when read together with the accompanying drawings. It should be understood that the drawings described below are for illustration purposes only and are not intended to limit the scope of the present teachings in any way.
FIG. 1A is a schematic illustration of exemplary AAV vector constructs for producing exogenous mRNAs encoding helper factors. FIG. 1B is a schematic illustration of exemplary AAV transfer vectors including genes of interest (GOI).
FIG. 2 is a schematic illustration of an exemplary method of producing recombinant lentiviral particles.
FIG. 3 is a schematic illustration of exemplary lentivirus constructs for producing exogenous mRNAs encoding helper factors and an exemplary lentivirus transfer vector including a gene of interest (GOI).

### DETAILED DESCRIPTION

The present invention provides, among other things, an improved method for producing recombinant viruses, such as lentiviruses or adeno-associated viruses, without the need for transfection or transduction of host cells with individual plasmids or viruses containing factors required for the cellular assembly of infectious viral particles. Rather, the present invention provides methods that use exogenous mRNAs (*e.g., in vitro* transcribed mRNAs) to supply helper factors needed for assembly of infectious viruses. Described in various embodiments herein are recombinant viral particles that include one or more gene of interest, methods of making such recombinant viral particles using exogenous mRNAs (*e.g., in vitro* transcribed mRNAs), and methods of using such recombinant viral particles.

Various aspects of the invention are described in detail in the following sections. The use of sections is not meant to limit the invention. Each section can apply to any aspect of the invention. In this application, the use of "or" means "and/or" unless stated otherwise.

### Definitions

In order for the present invention to be more readily understood, certain terms are first defined below. Additional definitions for the following terms and other terms are set forth throughout the specification.
*A or an:* The articles "a" and "an" are used herein to refer to one or to more than one (*i.e.,* to at least one) of the grammatical object of the article. By way of example, "an element" means one element or more than one element.
*Adeno-associated virus:* As used herein, the term "adeno-associated virus" or "AAV" includes, but is not limited to, AAV type 1, AAV type 2, AAV type 3 (including types 3A and 3B), AAV type 4, AAV type 5, AAV type 6, AAV type 7, AAV type 8, AAV type 9, AAV type 10, AAV type 11, avian AAV, bovine AAV, canine AAV, equine AAV, and ovine AAV (see, *e.g.,* Fields et al., Virology, volume 2, chapter 69 (4th ed., Lippincott-Raven Publishers); Gao et al., J. Virology 78:6381-6388 (2004); Mori et al., Virology 330:375-383 (2004)). Typically, AAV can infect both dividing and non-dividing cells and can be present in an extrachromosomal state without integrating into the genome of a host cell. AAV vectors are commonly used in gene therapy.
*Approximately or about:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value). Any numerals used in this application with or without about/approximately are meant to cover any normal fluctuations appreciated by one of ordinary skill in the relevant art.
*Coupled, linked, joined, or fused:* As used herein, the terms "coupled", "linked", "joined", "fused", and "fusion" are used interchangeably. These terms refer to the joining together of two more elements or components by whatever means, including chemical conjugation or recombinant means.
*Exogenous mRNA:* As used herein, "exogenous mRNA" means any mRNA that is introduced into an organism or cell and that is not synthesized by the recipient organism or cell itself. An exogenous mRNA can be isolated or purified from an organism or cell, can be transcribed *in vitro,* or can be produced by synthetic means. Exogenous mRNA does not include an RNA transcribed inside a recipient cell or organism, such as a packaging cell. In particular, exogenous mRNA does not include an mRNA transcribed or produced by a recipient cell or organism, such as a packaging cell, whether from an endogenous DNA or from an exogenous DNA (e.g., from a plasmid or virus vector) supplied to the recipient cell or organism.
*Expression control sequence:* As used herein, the term "expression control sequence" refers to a nucleic acid sequence that regulates the expression of a nucleotide sequence to which it is operably linked. An expression control sequence is "operably linked" to a nucleotide sequence when the expression control sequence controls and regulates the transcription and/or the translation of the nucleotide sequence. Thus, an expression control sequence typically includes promoters, enhancers, internal ribosome entry sites (IRES), transcription terminators, a start codon in front of a protein-encoding gene, splicing signal for introns, and/or stop codons. The term "expression control sequence" is intended to include, at a minimum, a sequence whose presence is designed to influence expression, and can also include additional advantageous components. For example, leader sequences and fusion partner sequences are expression control sequences. The term can also include the design of the nucleic acid sequence such that undesirable, potential initiation codons in and out of frame, are removed from the sequence. It can also include the design of the nucleic acid sequence such that undesirable potential splice sites are removed. It includes sequences or polyadenylation sequences (pA) which direct the addition of a polyA tail (*i.e.,* a string of adenine residues at the 3'-end of an mRNA), sequences referred to as polyA sequences. It also can be designed to enhance mRNA stability.
*gag, pol, or env protein*: As used herein, the terms "gag protein", "pol protein", and "env protein" refer to the multiple proteins encoded by retroviral gag, pol and env genes. As non-limiting examples, HIV gag encodes, among other proteins, p17, p24, p9 and p6. HIV pol encodes, among other proteins, protease (PR), reverse transcriptase (RT) and integrase (IN). HIV env encodes, among other proteins, Vpu, gp120 and gp41.
*Helper factor:* As used herein, "helper factor" refers to a viral protein involved in the replication of a viral genome and/or encapsidation of a viral particle. Typically, a helper factor is a viral enzyme, structural protein or a factor otherwise required for the assembly of virus. A helper factor can be supplied in trans for assembly of an infectious viral particle. In the case of lentiviruses, helper factors can include the Pol, Gag, Env, Tat, and/or Rev proteins. In the case of adeno-associated viruses, helper factors can include various Rep and Cap proteins.
*Heterologous nucleotide sequence:* The terms "heterologous nucleotide sequence" and "heterologous nucleic acid" are used interchangeably herein and refer to a sequence that is not naturally occurring in a virus. For example, a heterologous nucleic acid can be a non-viral promoter or an open reading frame that encodes a gene or nontranslated RNA of interest (e.g., for delivery to a cell or subject).
*Packaging cell:* As used herein, the term "packaging cell" generally refers to a cell that is used to produce recombinant viruses. Typically, a packaging cell contains or supplies elements such as helper factors required for production of infectious recombinant virus. For example, packaging cells can express viral structural proteins, enzymes, after being transfected with *in vitro* transcribed mRNAs encoding such viral structural proteins or enzymes.
*Packaging signal:* The term "packaging signal" is used interchangeably with "packaging sequence" or "psi" and refers to a non-coding, cis-acting sequence required for encapsidation of retroviral RNA strands during viral particle assembly.
*Promoter*: As used herein, the term "promoter" refers to a sequence of DNA, usually upstream (5') of the coding region of a gene, which controls the expression of the coding region by providing recognition and binding sites for RNA polymerase and other factors that may be required for initiation of transcription. Promoters are well known in the art. Exemplary promoters include the SV40, CMV and human elongation factor (EFI) promoters. Other suitable promoters are readily available in the art (see, *e.g.,* Ausubel et al., Current Protocols in Molecular Biology, John Wiley & Sons, Inc., New York (1998); Sambrook et al., Molecular Cloning: A Laboratory Manual, 2nd ed., Cold Spring Harbor University Press, New York (1989); and U.S. Pat. No. 5,681,735)).
*Purified:* By "purified" (or "isolated") refers to a nucleic acid sequence (*e.g.,* a polynucleotide) or an amino acid sequence (*e.g.,* a polypeptide) that is removed or separated from other components present in its natural environment. For example, an isolated polypeptide is one that is separated from other components of a cell in which it was produced (*e.g.,* the endoplasmic reticulum or cytoplasmic proteins and RNA). An isolated polynucleotide is one that is separated from other nuclear components (*e.g*., histones) and/or from upstream or downstream nucleic acid sequences. An isolated nucleic acid sequence or amino acid sequence can be at least 60% free, or at least 75% free, or at least 90% free, or at least 95% free from other components present in natural environment of the indicated nucleic acid sequence or amino acid sequence.
*Polynucleotide:* As used herein, "polynucleotide" (or "nucleotide sequence" or "nucleic acid molecule") refers to an oligonucleotide, nucleotide, or polynucleotide, and fragments or portions thereof, and to DNA or RNA of genomic or synthetic origin, which may be single- or double- stranded, and represent the sense or anti-sense strand.
*Substantial identity*: The phrase "substantial identity" is used herein to refer to a comparison between amino acid or nucleic acid sequences. As will be appreciated by those of ordinary skill in the art, two sequences are generally considered to be "substantially identical" if they contain identical amino acid residues or nucleotides in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul et al., Nucleic Acids Res. 25:3389-3402, 1997; Baxevanis et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying identical sequences, the programs mentioned above typically provide an indication of the degree of identity. In some embodiments, two sequences are considered to be substantially identical if at least 50%, 55%, 60%, 65%, 70%, 75%, 80%, 85%, 90%, 91%, 92%, 93%, 94%, 95%, 96%, 97%, 98%, 99% or more of their corresponding amino acid residues or nucleotides are identical over a relevant stretch of residues. In some embodiments, the relevant stretch is a complete sequence. In some embodiments, the relevant stretch is at least 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 or more amino acid residues or nucleotides.
*Pharmaceutically effective amount:* The term "pharmaceutically effective amount" or "therapeutically effective amount" refers to an amount (*e.g*., dose) effective in treating a patient, having a disorder or condition described herein. It is also to be understood herein that a "pharmaceutically effective amount" may be interpreted as an amount giving a desired therapeutic effect, either taken in one dose or in any dosage or route, taken alone or in combination with other therapeutic agents.
*Treatment:* As used herein, the term "treatment" (also "treat" or "treating") refers to any administration of a therapeutic gene (e.g., gene encoding a lysosomal enzyme) that partially or completely alleviates, ameliorates, relieves, inhibits, delays onset of, reduces severity of and/or reduces incidence of one or more symptoms or features of a particular disease, disorder, and/or condition (e.g., a lysosomal storage disease). Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition.
*Subject:* As used herein, the term "subject" means any mammal, including humans. In certain embodiments of the present invention the subject is an adult, an adolescent or an infant. Also contemplated by the present invention are the administration of the pharmaceutical compositions and/or performance of the methods of treatment in-utero.
*Transformation:* As used herein, the term "transformation" generally refer to the introduction of an exogenous nucleic acid, *e.g.,* an mRNA molecule or an expression vector, into a recipient cell by direct taking up through the cell membranes. In some embodiments, the term "transduction" is used to describe the introduction of exogenous nucleic acid into host cells by viral vectors. In some embodiments, the term "transfection" is used to describe the introduction of exogenous nucleic acids into animal cells.
*Transfer vector:* The term "transfer vector", as used herein, refers to a plasmid comprising a gene of interest and viral cis-acting sequences required for packaging, reverse transcription, and integration. A transfer vector can be used to introduce a gene of interest into a packaging cell.
*Virus vector*: As used herein, the term "virus vector" refers to a virus (*e.g.,* lentivirus or AAV) based vector that functions as a nucleic acid delivery vehicle. Typically, a virus vector contains a gene of interest and at least a portion of viral genome that facilitates virus infection and/or integration. In some embodiments, a viral vector is packaged and delivered within a viral particle.

### Retroviruses

The methods described herein can be used to produce recombinant retroviral particles, including, but not limited to, viral particles of murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other retroviridiae including lentiviruses. See, *e.g.,* Coffin et al., "Retroviruses", 1997 Cold Spring Harbor Laboratory Press Eds: J M Coffin, S M Hughes, H E Varmus, pp 758-763). In some embodiments, the present invention is used to produce a retrovirus that is not moloney murine leukemia virus.

In some embodiments, the present invention is used to produce a lentivirus. Lentiviruses are retroviruses that can infect both dividing and non-dividing cells (see, *e.g.,* Lewis et al., EMBO J. 3053-3058 (1992)). Typically, lentiviruses can be split into "primate" and "non-primate". Non-limiting examples of primate lentiviruses include human immunodeficiency virus (HIV) and simian immunodeficiency virus (SIV). The non-primate lentiviral group includes the prototype "slow virus" visna/maedi virus (VMV), caprine arthritis-encephalitis virus (CAEV), equine infectious anaemia virus (EIAV), feline immunodeficiency virus (FIV), and bovine immunodeficiency virus (BIV). The genomic sequences of lentiviruses are known in the art (see, *e.g*., Genbank Accession Nos. AF033819 and AF033820, the HIV databases maintained by Los Alamos National Laboratory, and the NCBI database maintained by the National Institutes of Health).

During the process of infection, a retrovirus initially attaches to a specific cell surface receptor. On entry into the susceptible host cell, the retroviral RNA genome is copied to DNA by a virally encoded reverse transcriptase, which is carried inside the parent virus. This DNA is transported to the host cell nucleus, where it subsequently integrates into the host genome. At this stage, it is typically referred to as the provirus. The provirus is stable in the host chromosome during cell division and is transcribed like other cellular genes. The provirus encodes the proteins and other factors required to make more virus, which can leave the cell by a process called "budding".

Each retroviral genome comprises genes called gag, pol and env, which code for virion proteins and enzymes. These genes are flanked at both ends by regions called long terminal repeats (LTRs). The LTRs are responsible for proviral integration, and transcription. They also serve as enhancer-promoter sequences and can control the expression of the viral genes. Encapsidation of the retroviral RNAs occurs by virtue of a psi sequence located at the 5' end of the viral genome. The LTRs are identical sequences that can be divided into three elements, which are called U3, R and U5. U3 is derived from the sequence unique to the 3' end of the RNA. R is derived from a sequence repeated at both ends of the RNA, and U5 is derived from the sequence unique to the 5' end of the RNA. The sizes of the three elements can vary considerably among different retroviruses.

For the viral genome, the site of transcription initiation is at the boundary between U3 and R in the left hand side LTR and the site of poly (A) addition (termination) is at the boundary between R and U5 in the right hand side LTR. U3 contains most of the transcriptional control elements of the provirus, which include the promoter and multiple enhancer sequences responsive to cellular and in some cases, viral transcriptional activator proteins. Some retroviruses have any one or more of the following genes that code for proteins that are involved in the regulation of gene expression: tat, rev, tax and rex.

The structural gene gag encodes the internal structural protein of the virus. Gag protein is proteolytically processed into the mature proteins MA (matrix), CA (capsid) and NC (nucleocapsid). The structural gene pol encodes a reverse transcriptase (RT), which contains DNA polymerase, associated RNase H and integrase (IN), which mediate replication of the genome. The structural gene env encodes the surface (SU) glycoprotein and the transmembrane (TM) protein of the virion, which form a complex that interacts specifically with cellular receptor proteins. This interaction leads ultimately to infection by fusion of the viral membrane with the cell membrane.

Retroviruses can also contain additional genes, which code for proteins other than Gag, Pol and Env. Examples of additional genes include in HIV, one or more of vif, vpr, vpx, vpu, tat, rev and nef. EIAV has, for example, the additional genes S2 and dUTPase. Proteins encoded by additional genes serve various functions. In EIAV, for example, tat acts as a transcriptional activator of the viral LTR. It binds to a stable, stem-loop RNA secondary structure referred to as TAR. Rev regulates and co-ordinates the expression of viral genes through rev-response elements (RRE). In addition, an EIAV protein, Ttm, has been identified that is encoded by the first exon of tat spliced to the env coding sequence at the start of the transmembrane protein. Further, sequences such as a central polypurine tract (cPPT) and/or central termination sequence (CTS), can be included (see, e.g., Riviere et al., J. Virol. 84:729-739 (2010)), e.g., to increase transduction efficiency. In some embodiments, nucleotides 4328-4451 from the HIV genome GenBank sequence AF033819, are included in a retroviral particle (e.g., in a transfer vector) described herein: 5'tttaaaagaaaaggggggattggggggtacagtgcaggggaaagaatagtagacataatagcaacagacatacaaactaaagaattac aaaaacaaattacaaaaattcaaaattttcgggttt3' (SEQ ID NO:1). In other embodiments, a sequence having at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or more, identity to SEQ ID NO:1 is included in a retroviral particle (e.g., in a transfer vector) described herein.

Lentiviral vectors and their use in gene transfer are reviewed in, *e.g*., Dropulic, Hum. Gene Ther. 22:649-57 (2011); and Kumar et al., Curr. Gene Ther. 11:144-53 (2011).

### Parvoviruses

The methods described herein can be used to produce recombinant parvoviral particles, including viral particles of dependoviruses such as infectious human or simian AAV, for the introduction and/or expression of nucleic acids in mammalian cells.

Viruses of the Parvoviridae family are small DNA animal viruses. The family Parvoviridae may be divided between two subfamilies: the Parvovirinae, which infect vertebrates, and the Densovirinae, which infect insects. Members of the subfamily Parvovirinae include the genus *Dependovirus.* The genus *Dependovirus* includes AAV, which normally infects humans (*e.g.,* serotypes 1, 2, 3A, 3B, 4, 5, and 6) or primates (*e.g.,* serotypes 1 and 4), and includes related viruses that infect other warm-blooded animals (*e.g*., bovine, canine, equine, and ovine adeno-associated viruses).

The genomic organization of known AAV serotypes is very similar. The genome of AAV is a linear, single-stranded DNA molecule that is less than about 5,000 nucleotides in length. Inverted terminal repeats (ITRs) flank the unique coding nucleotide sequences for the non-structural replication (Rep) proteins and the structural (VP) proteins. The VP proteins form the capsid. Thus, the genes encoding the VP proteins are also referred to as *cap* genes. The terminal 145 nucleotides are self-complementary and are organized so that an energetically stable intramolecular duplex forming a T-shaped hairpin may be formed. These hairpin structures function as an origin for viral DNA replication, serving as primers for the cellular DNA polymerase complex. In general, the *rep* genes encode the Rep proteins, Rep78, Rep68, Rep52, and Rep40. The genes rep78 and rep68 are typically transcribed from the p5 promoter, and rep52 and rep40 are typically transcribed from the p19 promoter. Typically, the *cap* genes encode the VP proteins, VP1, VP2, and VP3. The *cap* genes are transcribed from the p40 promoter. An additional protein involved in capsid formation is Assembly Activating Protein (AAP) (see, e.g., Sonntag et al., PNAS 107:10220-10225 (2010)).

The genomic sequences of various serotypes of AAV, as well as the sequences of the native terminal repeats (TRs), Rep proteins, and capsid subunits, are known in the art. See, *e.g.,* GenBank Accession Numbers NC_002077, NC_001401, NC_001729, NC_001829, NC_006152, NC_006261, AF063497, U89790, AF043303, AF028705, AF028704, J01901, AF513851, AF513852, AF085716, and AY530579; see also, *e.g.,* Srivistava et al., J. Virology 45:555-564 (1983); Chiorini et al., J. Virology 71:6823-6833 (1998); Chiorini et al., J. Virology 73:1309-1319 (1999); Bantel-Schaal et al., J. Virology 73:939-947 (1999); Xiao et al., J. Virology 73:3994-4003 (1999); Muramatsu et al., Virology 221:208-217 (1996); Gao et al., Proc. Nat. Acad. Sci. USA 99:11854-11859 (2002); Mori et al., Virology 330:375-383 (2004); international patent publications WO 00/28061, WO 99/61601, WO 98/11244; and U.S. Pat. No. 6,156,303.

The capsid structures of AAV are described in more detail in Fields et al., Virology, volume 2, chapters 69 & 70 (4th ed., Lippincott-Raven Publishers); Xie et al., Proc. Nat. Acad. Sci. 99:10405-10 (2002); Padron et al., J. Virol. 79: 5047-58 (2005); Walters et al., J. Virol. 78: 3361-71(2004); Xie et al., J. Mol. Biol. 6:497-520 (1996); and Tsao et al., Science 251:1456-64 (1991).

AAV vectors and their use in gene transfer applications are reviewed in, *e.g.,* Ayuso et al., Curr. Gene Ther. 10:423-436 (2010); and Bueler, Biol. Chem. 380:613-622 (1999).

### Exogenous mRNA Molecules

Inventive methods described herein utilize exogenous mRNAs to produce viral particles. In particular, exogenous mRNAs encoding one or more helper factors are directly introduced to packaging cells for assembly of recombinant viruses. Helper factors for assembly of a retroviral particle (e.g., a lentiviral particle) can include the Pol, Gag, Env, Tat, Rev proteins and/or other structural proteins or enzymes as described herein. In the case of adeno-associated viruses, helper factors can include various Rep, Cap/VP, and AAP proteins described herein.

Methods of obtaining or producing exogenous mRNA molecules are known in the art, and any such method can be used to obtain mRNA molecules for use in the present invention. For example, suitable exogenous mRNAs can be transcribed from a DNA template (e.g., genomic DNA, plasmid DNA, phage DNA, cDNA, synthetic DNA sequence or any other appropriate source of DNA) encoding desired helper factors. Exogenous mRNAs may be transcribed *in vitro* from DNA templates encoding desired helper factors, may be produced by and isolated from cells containing desired DNA templates, or can be produced synthetically using known cDNA sequences. Sequences encoding helper factors to be transcribed into exogenous mRNAs can be obtained from any known source, including lentiviral genomic RNA, or cDNAs corresponding to viral RNA. Suitable cDNAs corresponding to lentiviral genomic RNA are commercially available and include, for example, pNLENV-1 (Maldarelli et al., J. Virol. 65:5732 (1991)), which contains genomic sequences of HIV-1. Retroviral (*e.g*., lentiviral) cDNA clones are also available from the American Type Culture Collection (ATCC), Rockville, Md. In addition, sequences for lentiviruses and AAVs are available from GenBank as described herein.

In some embodiments, DNA templates are configured such that, once transcribed, the one or more helper factors are encoded by one single exogenous mRNA molecule. In some embodiments, the one or more helper factors are encoded by two or more exogenous mRNA molecules. In some embodiments, individual helper factor is encoded by separate individual exogenous mRNA molecule. When two or more helper factors are encoded by a single mRNA, an internal ribosome entry sites (IRES) is typically designed between sequences encoding different helper factors to facilitate translation of multiple helper factors from a single mRNA.

In some methods, mRNA is synthesized *in vitro* from a desired DNA template (*e.g.,* genomic DNA, plasmid DNA, phage DNA, cDNA, synthetic DNA sequence or any other appropriate source of DNA). For example, mRNAs can be *in vitro* transcribed from T7 or similar viral promoters like T3 and SP6. Various in vitro RNA transcription systems are known in the art and can be used to practice the present invention.

mRNA molecules can be modified to increase translation and/or stability (see, *e.g.,* U.S. Publ. No. 20110077287). For example, mRNA can be synthesized to include a cap on the 5' end and/or a 3' poly(A) tail, which determine ribosome binding, initiation of translation and stability mRNA in the cell. In particular, the 5' cap and/or poly(A) tail can facilitate translation such that upon entry into a packaging cell, exogenous mRNA sequences are translated within the cytoplasm of the packaging cells to produce the encoded proteins. Methods of adding 5' caps and poly(A) tails are known in the art (see, *e.g.,* Cougot et al., Trends in Biochem. Sci. 29:436-444 (2001); Stepinski et al., RNA 7:1468-95 (2001); Elango et al., and Biochim. Biophys. Res. Commun. 330:958-966 (2005); Yokoe et al., Nature Biotechnology. 1996 14:1252-1256)). mRNA molecules can also contain modified nucleotides, sugar or phosphate group to resist degradation and increase stability. In addition, mRNA can be stabilized using known stabilizing reagents such as, but not limited to, proteins, peptides, aptamers, translational accessory proteins, mRNA binding proteins, and/or translation initiation factors (see, *e.g.,* US Pat. No. 5,677,124). Additional known methods of stabilizing the mRNA include, e.g., the inclusion of a Kozac consensus sequence (Kozac, Nucleic Acids Res. 15:8125-8148 (1987)), codon optimization (Heidenreich et al., J. Biol. Chem. 269:2131-2138 (1994)), the inclusion of pseudouridines (Kariko et al., Mol. Ther. 16:1833-1840 (2008)), hybridization to complementary nucleic acid molecules (Krieg et al., Methods Enzymology 155:397-415 (1987)), the use of lipids (Lasic, Trends Biotechnol. 16:307-321 (1998); Lasic et al., FEBS Lett. 312:255-258 (1992)), the use of polycations (Caplen et al., Gene Ther. 2:603 (1995); Li et al., Gene Ther. 4:891 (1997)), and the use of locked nucleic acids (LNA) (Tolstrup et al., Nucleic Acids Res. 31:3758-3762 (2003); and Exiqon A/S, Vedbaek, Denmark)). Additional methods of modifying exogenous mRNA molecules are described in PCT Appln. No. PCT/US2012/041724.

An exogenous mRNA molecule can be introduced into packaging cells using any method. Exemplary methods include, without limitation, microinjection, electroporation, lipid-mediated transfection (e.g., lipofectamine, cationic liposomes), or poly(ethylenimine) (PEI) transfection.

In certain embodiments, one or more or all exogenous mRNAs are supplied, e.g., transfected, at the same or about the same level. In other embodiments, two or more exogenous mRNAs are supplied, e.g., transfected, at different levels. For example, in the case of lentivirus, the ratio of mRNA encoding a Gag protein to mRNA encoding a Pol protein is about 50:1, 45:1, 40:1, 35:1, 30:1, 25:1, 20:1, 15:1, 10:1, 5:1, or 1:1. In the case of AAV, the ratio of mRNA encoding a Rep78 protein to mRNA encoding a Rep52 protein is about 50:1, 45:1, 40:1, 35:1, 30:1, 25:1, 20:1, 15:1, 10:1, 5:1, or 1:1.

### Methods of Producing Viral Particles

The present invention provides methods of producing recombinant viruses, in particular, lentiviruses or AAVs, without the need for transfection or transduction of packaging cells with individual plasmids or viruses encoding helper factors required for the cellular assembly of infectious viruses, *e.g*., containing a therapeutic gene of interest. Rather, inventive methods described herein supply one or more helper factors by exogenous mRNAs.

### AAV Particles

In some embodiments, the present invention provides methods of producing recombinant AAV particles based on the use of exogenous mRNAs. For example, one or more exogenous mRNAs encoding AAV helper factors (*e.g*., AAV Rep 78, Rep 52, AAP, VP1, VP2, and/or VP3 proteins) are introduced into packaging cells (*e.g*., mammalian cells) for assembly of AAV particles with a nucleic acid typically containing a gene of interest. AAV helper factors can be encoded by a single mRNA molecule. In some embodiments, one or more helper factors can be encoded by multiple separate mRNA molecules. In that case, multiple mRNA molecules are co-transfected into packaging cells.

Typically, packaging cells include a nucleotide sequence that comprises a gene of interest and AAV cis-acting sequences (as described herein) required for packaging, reverse transcription and integration, or both (*e.g.,* a packaging signal, *e.g.,* a psi). In some instances, a suitable AAV nucleotide sequence is provided in a vector or plasmid (referred to as "transfer vector"), and the packaging cell is transiently co-transfected with (i) a transfer vector and (ii) one or more exogenous mRNAs encoding one or more AAV helper factors (*e.g*., AAV Rep 78, Rep 52, AAP, VP1, VP2, and/or VP3). In other instances, a packaging cell is stably transfected with a transfer vector, and the gene of interest is integrated into the genome of the packaging cell.

Virus stocks are produced by maintaining the packaging cells (e.g., transfected with exogenous mRNAs and optionally co-transfected with a transfer vector) under conditions suitable for virus production (e.g., in an appropriate growth media and for an appropriate period of time). Such conditions are not limiting, and are generally known in the art (see, *e.g.,* Sambrook et al., Molecular Cloning: A Laboratory Manual, 2nd Ed., Cold Spring Harbor University Press, New York (1989); Ausubel et al., Current Protocols in Molecular Biology, John Wiley & Sons, New York (1998); U.S. Pat. No. 5,449,614; and U.S. Pat. No. 5,460,959).

In one representative method, the method includes introducing into a packaging cell described herein: (i) a transfer vector comprising a gene of interest and at least one ITR sequence (*e.g.,* AAV ITR sequence), and (ii) exogenous mRNAs encoding AAV helper factors sufficient for replication of the gene of interest and encapsidation into AAV particles (*e.g*., AAV Rep 78, Rep 52, AAP, VP1, VP2, and/or VP3 proteins). In particular embodiments, a transfer vector comprises two AAV ITR sequences, which are located 5' and 3' to a gene of interest.

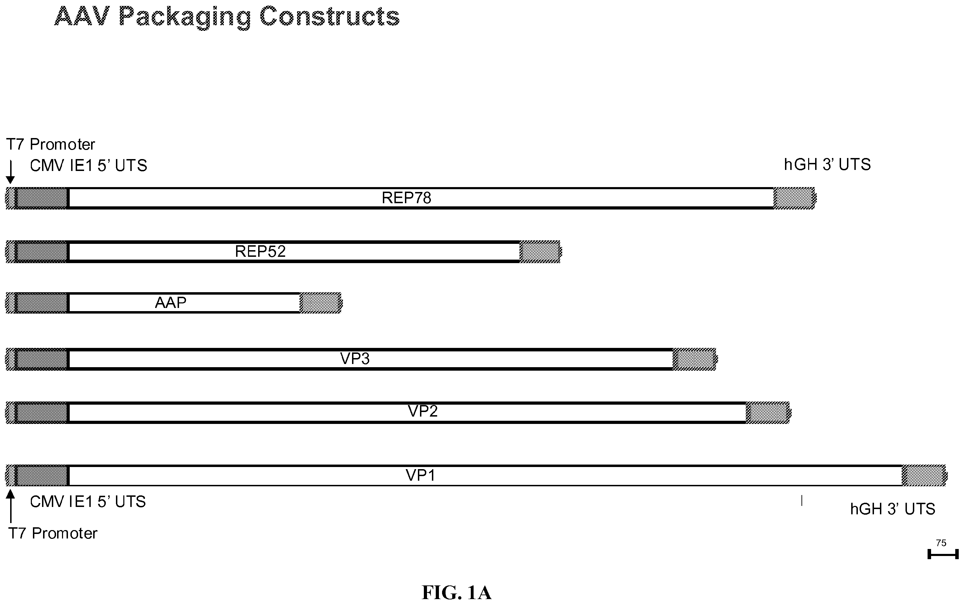

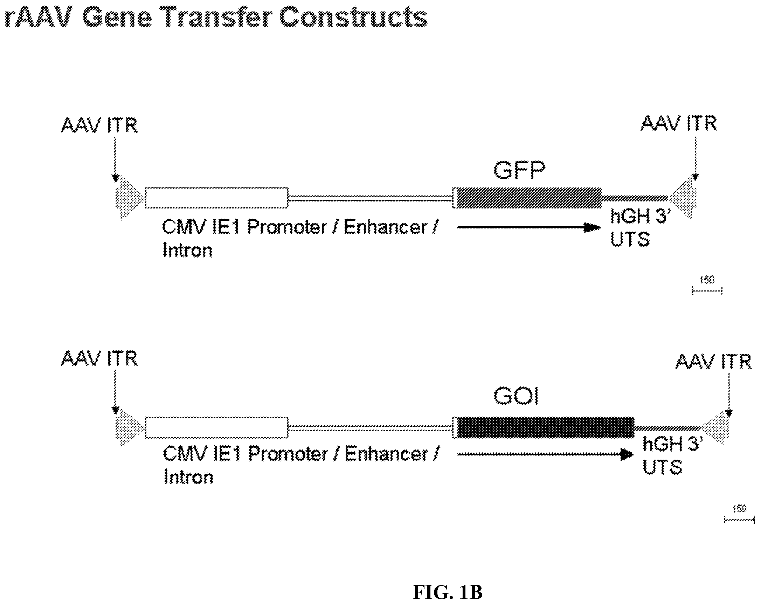

Exemplary constructs for producing exogenous mRNAs described herein are depicted in Figure 1A. As shown in Figure 1A, nucleic acids encoding helper factors Rep78, Rep52, AAP, VP1, VP2, and VP3 are included in vectors that include a T7 promoter, CMV IE1 5' UTS, and hGH 3' UTS. Figure 1B depicts exemplary transfer vectors comprising either a gene encoding GFP marker protein or a gene of interest (GOI). The exemplary transfer vectors shown in Figure 1B include an AAV ITR sequence, a CMV IE1 promoter/enhancer/intron sequence, a gene of interest, a hGH 3' UTS, and a second AAV ITR sequence.

### Lentiviral Particles

In some embodiments, the present invention provides methods of producing recombinant lentiviral particles based on the use of exogenous mRNAs. For example, one or more exogenous mRNAs encoding lentiviral helper factors (*e.g*., lentiviral Pol, Gag, and Env proteins) are introduced into packaging cells (*e.g*., mammalian cells) for assembly of lentiviral particles with a nucleic acid typically containing a gene of interest. Again, lentiviral helper factors can be encoded by a single mRNA molecule. In some embodiments, one or more helper factors can be encoded by multiple separate mRNA molecules. In that case, multiple mRNA molecules are co-transfected into packaging cells.

A packaging cell can include a lentiviral nucleotide sequence that comprises a gene of interest and lentiviral cis-acting sequences (as described herein) required for packaging, reverse transcription and integration, or both (*e.g.,* a packaging signal, *e.g.,* a psi). In some instances, the lentiviral nucleotide sequence is provided in a vector or plasmid (also referred to as "transfer vector"), and the packaging cell is transiently co-transfected with (i) a transfer vector and (ii) one or more exogenous mRNAs encoding one or more lentiviral helper factors (*e.g*., lentiviral Pol, Gag, and Env). In other instances, a packaging cell is stably transfected with a transfer vector, and the gene of interest is integrated into the genome of the packaging cell.

Virus stocks are produced by maintaining the packaging cells (*e.g*., transfected with exogenous mRNAs and optionally co-transfected with a transfer vector) under conditions suitable for virus production (*e.g*., in an appropriate growth media and for an appropriate period of time). Such conditions are not limiting, and are generally known in the art (see, *e.g.,* Sambrook et al., Molecular Cloning: A Laboratory Manual, 2nd Ed., Cold Spring Harbor University Press, New York (1989); Ausubel et al., Current Protocols in Molecular Biology, John Wiley & Sons, New York (1998); U.S. Pat. No. 5,449,614; and U.S. Pat. No. 5,460,959).

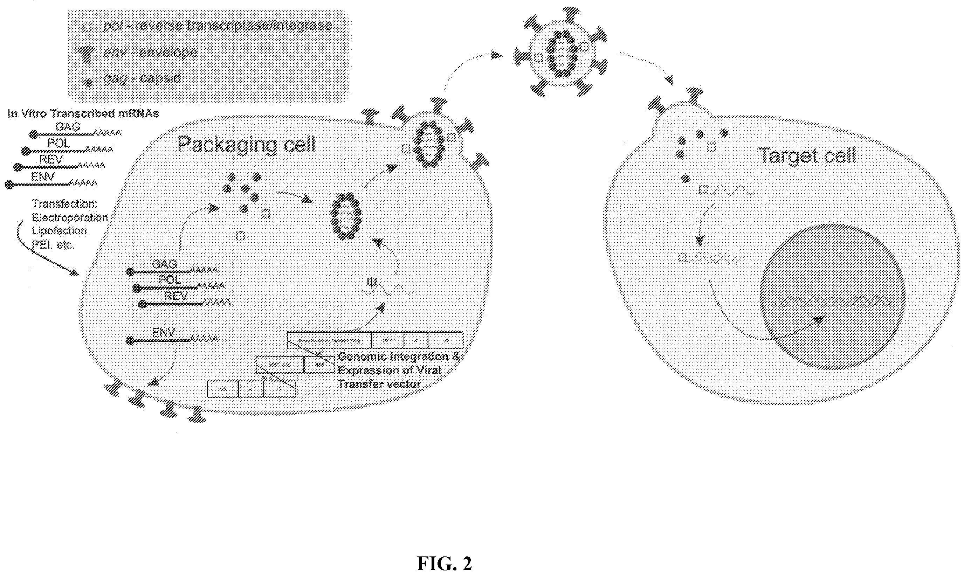

Figure 2 depicts one exemplary method of producing a recombinant lentiviral particle according to an inventive method described herein. As shown in Figure 2, *in vitro* transcribed mRNAs are introduced into a packaging cell. The mRNAs encode lentiviral Gag, Pol, Rev, and Env proteins, which are produced by translation of the mRNAs in the cytoplasm of the packaging cell. As depicted in Figure 2, the packaging cell also includes a viral transfer vector integrated within its genome. The viral transfer vector includes a packaging signal (ψ), and upon expression of the viral transfer vector, the viral RNA genome (which includes RNA encoded by a gene of interest) is encapsulated within a viral particle, which leaves the packaging cell by budding. The viral particle can then bind to and enter a target cell, where the viral RNA genome is copied to DNA by a virally encoded reverse transcriptase carried by the viral particle. This DNA is then transported to the host cell nucleus, where it subsequently integrates into the host genome.

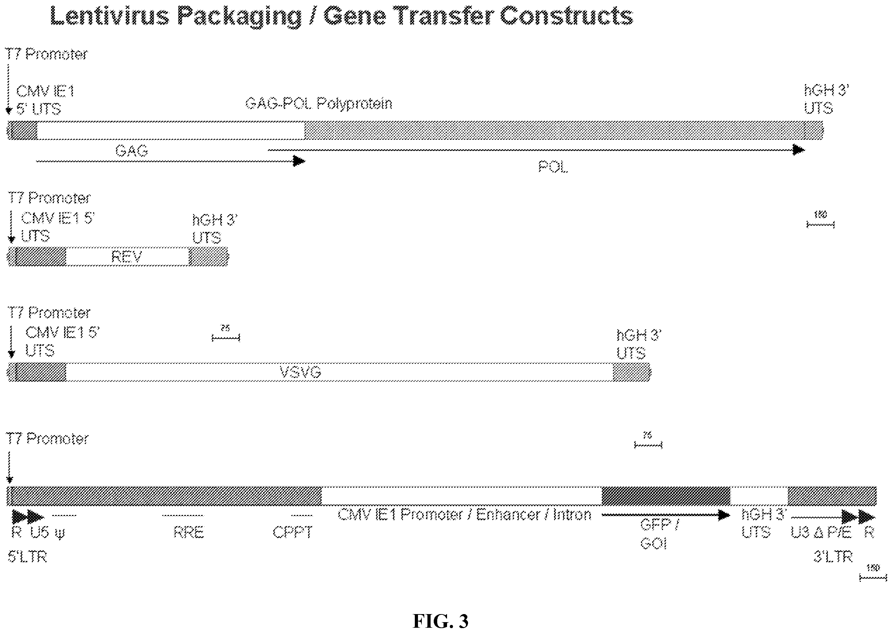

Figure 3 depicts exemplary lentivirus vector constructs for producing exogenous mRNAs encoding lentivirus helper factors and also depicts an exemplary lentivirus transfer vector including a gene of interest. As shown in Figure 3, nucleic acids encoding helper factors Gag, Pol, Rev, and VSV-G are included in vectors that include a T7 promoter, CMV IE 1 5' UTS, and hGH 3' UTS. The exemplary transfer vector shown in Figure 3 includes a T7 promoter, a 5' LTR, a ψ packaging signal, RRE, CPPT (central polypurine tract), and CMV IE1 promoter/enhancer/intron sequences, a gene of interest, a hGH 3' UTS, and a 3' LTR.

In another representative method, the method includes introducing into a packaging cell described herein: (i) a transfer vector comprising a gene of interest and at least one LTR sequence (*e.g*., lentiviral LTR sequence), and (ii) exogenous mRNAs encoding lentiviral proteins sufficient for replication of the gene of interest and encapsidation into lentiviral particles (*e.g*., lentiviral Pol, Gag, and Env proteins). In particular embodiments, the transfer vector comprises two lentiviral LTR sequences, which are located 5' and 3' to a gene of interest.

Suitable retroviral sequences that can be used in the methods described herein can be obtained from commercially available sources. For example, such sequences can be purchased in the form of retroviral plasmids, such as pLJ, pZIP, pWE and pEM. Suitable packaging sequences that can be employed in the vectors described herein are also commercially available including, for example, plasmids ψCrip, ψCre, ψ2 and ψAm.

### Pseudotyping

The structural envelope proteins (e.g., Env, VP1, VP2, or VP3) can determine the range of host cells that can ultimately be infected and transformed by recombinant viruses. In the case of lentiviruses, such as HIV-1, HIV-2, SIV, FIV and EIV, the Env proteins include gp41 and gp120.

When producing recombinant retroviruses (*e.g*., recombinant lentiviruses or AAVs), a wild-type viral (e.g., lentiviral or AAV)) env, vp1, vp2, or vp3 gene can be used, or can be substituted with any other viral env, vp1, vp2, or vp3 gene from another lentivirus or AAV or other virus (such as vesicular stomatitis virus GP (VSV-G)). Methods of pseudotyping recombinant viruses with envelope proteins from other viruses in this manner are well known in the art (see, *e.g.,* WO 99/61639, WO 98/05759, Mebatsion et al., Cell 90:841-847 (1997); Cronin et al., Curr. Gene Ther. 5:387-398 (2005)).

### Genes of Interest

In certain instances, a gene of interest is incorporated into the genome of a recombinant viral particle. A gene of interest can include any suitable nucleotide sequence, such as all or a portion of a naturally occurring DNA or RNA sequence. A gene of interest can be, for example, a synthetic RNA/DNA sequence, a codon optimized RNA/DNA sequence, a recombinant RNA/DNA sequence (*i.e*., prepared by use of recombinant DNA techniques), a cDNA sequence, or a partial genomic DNA sequence, including combinations thereof. The gene of interest can be a coding region or a noncoding region. In addition, an RNA/DNA sequence can be in a sense orientation or in an anti-sense orientation.

A gene of interest is also referred to herein as a "heterologous sequence", "heterologous gene" or "transgene". A gene of interest can be, *e.g.,* a selection gene, marker gene, or therapeutic gene. As described herein, the gene of interest can be included within a transfer vector that further includes cis-acting viral packaging sequences.

In some embodiments, a gene of interest is operably linked with one or more control sequences that can regulate and/or drive gene expression. Exemplary control sequences include, but are not limited to, a transcriptional promoter, enhancer, suppressor, insulator, an optional operator sequence to control transcription of an operon, a sequence encoding suitable mRNA ribosomal binding sites, and sequences that control termination of transcription and translation. In a particular instance, a gene of interest can be placed under the regulatory control of a promoter that can be induced or repressed, thereby offering a greater degree of control with respect to the level of expression.

A gene of interest can be isolated from natural sources or be produced recombinantly or synthetically. A gene of interest can contain naturally-occurring sequences or modified sequences, or manufactured *de novo,* as described in, for example, Ausubel et al., Current Protocols in Molecular Biology, John Wiley & Sons, New York (1998); and Sambrook et al., Molecular Cloning: A Laboratory Manual, 2nd ed., Cold Spring Harbor University Press, New York. (1989). More than one gene of interest can be fused, linked or coupled together by methods known in the art, such as by exploiting and manufacturing compatible cloning or restriction sites.

A gene of interest can have a therapeutic or diagnostic application. Suitable genes of interest include, but are not limited to, sequences encoding enzymes, cytokines, chemokines, hormones, antibodies, anti-oxidant molecules, engineered immunoglobulin-like molecules, single chain antibodies, fusion proteins, immune co-stimulatory molecules, immunomodulatory molecules, antisense RNA, small interfering RNA (siRNA), a transdominant negative mutant of a target protein, a toxin, a conditional toxin, an antigen, a tumour suppresser protein, growth factors, membrane proteins, pro- and anti-angiogenic proteins and peptides, vasoactive proteins and peptides, anti-viral proteins and ribozymes, and derivatives thereof (such as with an associated reporter group). Genes of interest can also encode pro-drug activating enzymes. Genes of interest can also encode reporters such as, but not limited to, green fluorescent protein (GFP), luciferase, beta-galactosidase, or resistance genes to antibiotics such as, for example, ampicillin, neomycin, bleomycin, zeocin, chloramphenicol, hygromycin, kanamycin, among others.

In some embodiments, the present invention is used to deliver a therapeutic gene in gene therapy for a disease, disorder or condition associated with deficiency of a target gene function. Thus, in some embodiments, a gene of interest encodes all or part of a target gene function. For example, a gene of interest can be identical to the target gene or a mutant, homolog or variant thereof. A gene of interest can also encode a fragment of a target protein that is capable of functioning *in vivo* in an analogous manner to the wild-type protein. The term "mutant" refers to a modified gene that encodes a modified protein with one or more amino acid variations (e.g., additions, deletions or substitutions) from the wild-type sequence. The term "homolog", as used herein, means an entity having a certain homology with the target protein, or that encodes a protein having a degree of homology with the target protein. A homologous sequence can be an amino acid sequence that is at least about 50%, 55%, 60%, 65%, 70%, 75%, 80%, 85%, 90%, 95%, 97%, 98%, 99%, or more, identical to a target protein.

A mutant can also have deletions, insertions or substitutions of amino acid residues that produce a silent change and result in a functionally equivalent substance. Deliberate amino acid substitutions can be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Viral particles described herein can include at least one, two or more genes of interest. For two or more genes of interest, two or more transcription units within the viral particle, one for each gene of interest, can be used. Alternatively, an internal ribosome entry site (IRES) can be used to initiate translation of the second (and subsequent) coding sequence(s) in a poly-cistronic message. Insertion of IRES elements into retroviral vectors is compatible with the retroviral replication cycle and allows expression of multiple coding regions from a single promoter (see, *e.g.,* Adam et al., J. Virol. 65:4985 (1991); Koo et al., Virology 186:669-675 (1992); Chen et al., J. Virol 67:2142-2148 (1993)).

Other methods of co-expressing multiple genes of interest from one viral particle include the use of multiple internal promoters in the vector, or the use of alternate splicing patterns leading to multiple RNA species derived from the single viral genome that expresses the different genes. See, *e.g.,* Overell et al., Mol. Cell Biol. 8:1803-8 (1988); Cepko et al., Cell 37:1053 (1984).

### Inclusion of Targeting Sequences

In some instances, the recombinant virus particle includes a targeting sequence (e.g., inserted into a viral helper factor, *e.g.,* a viral envelope or caspid protein). The targeting sequence can direct the viral particle to interact with a target cell or target tissue *(e.g.,* to interact with one or more cell-surface molecules present on a target cell or target tissue). The use of targeting sequences on viral particles is known in the art (see, *e.g*., international patent publication WO 00/28004; Hauck et al., J. Virology 77:2768-2774 (2003); Shi et al., Human Gene Therapy 17:353-361 (2006); and Grifman et al., Mol. Ther. 3:964-975 (2001)).

In some instances, one or more non-naturally occurring amino acids are incorporated into a viral particle capsid subunit at an orthogonal site as a means of redirecting a low-transduction viral particle to a desired target tissue (see, *e.g.,* Wang et al., Ann. Rev. Biophys. Biomol. Struct. 35:225-49 (2006)). Such amino acids can be used to chemically link targeting molecules to the capsid protein. Methods of chemically modifying amino acids are known in the art (see, *e.g*., Hermanson, Bioconjugate Techniques, 1st ed., Academic Press, 1996).

Exemplary targeting sequences include ligands and other peptides that bind to cell surface receptors and glycoproteins, such as RGD peptide sequences, bradykinin, hormones, peptide growth factors (*e.g*., epidermal growth factor, nerve growth factor, fibroblast growth factor, platelet-derived growth factor, insulin-like growth factors I and II, *etc*.), cytokines, melanocyte stimulating hormone (*e.g*., alpha, beta, or gamma), neuropeptides and endorphins, and the like, and fragments thereof that retain the ability to target to their cognate receptors. Other exemplary peptides and proteins include substance P, keratinocyte growth factor, neuropeptide Y, gastrin releasing peptide, interleukin 2, hen egg white lysozyme, erythropoietin, gonadoliberin, corticostatin, beta-endorphin, leu-enkephalin, rimorphin, alpha-neo-enkephalin, angiotensin, pneumadin, vasoactive intestinal peptide, neurotensin, motilin, and fragments thereof. The targeting sequence can target a cell surface binding site, including receptors (*e.g*., protein, carbohydrate, glycoprotein or proteoglycan). Specific, nonlimiting examples of cell surface binding sites include heparan sulfate, chondroitin sulfate, and other glycosaminoglycans, sialic acid moieties found on mucins, glycoproteins, and gangliosides, MHC I glycoproteins, carbohydrate components found on membrane glycoproteins, including, mannose, N-acetylgalactosamine, N-acetyl-glucosamine, fucose, galactose, and the like.

In other instances, the targeting sequence is a binding domain from a toxin (e.g., tetanus toxin or snake toxins, such as alpha-bungarotoxin, and the like). In other instances, the capsid protein can be modified by substitution of a "nonclassical" import/export signal peptide (*e.g.,* fibroblast growth factor-1 and -2, interleukin 1, HIV-1 Tat protein, herpes virus VP22 protein, and the like) into the capsid protein (see, *e.g.,* Cleves, Current Biology 7:R318 (1997)). Additional peptides include peptide motifs that direct uptake by specific cells (such as a FVFLP peptide motif to trigger uptake by liver cells).

### Packaging Cells

Various cell lines can be used as packaging cells according to the present invention. Generally, packaging cells are mammalian cells, such as human cells. Suitable mammalian cells include, without limitation, human (such as HT-1080, HeLa, 293, NIH 3T3), bovine, ovine, porcine, murine, hamster (such as CHO and BHK), rabbit and monkey (such as COS cells). A packaging cell may be a non-dividing cell (including hepatocytes, myofibers, hematopoietic stem cells, neurons) or a dividing cell. A packaging cell may be an embryonic cell, bone marrow stem cell or other progenitor cell. Where the cell is a somatic cell, the cell can be, for example, an epithelial cell, fibroblast, smooth muscle cell, blood cell (including a hematopoietic cell, red blood cell, T-cell, B-cell, *etc*.), tumor cell, cardiac muscle cell, macrophage, dendritic cell, neuronal cell (*e.g.,* a glial cell or astrocyte), or pathogen-infected cell (*e.g.,* those infected by bacteria, viruses, virusoids, parasites, or prions). Other suitable packaging cell lines for use in the methods described herein include other human cell line derived packaging cell lines and murine cell line derived packaging cell lines, such as Psi-2 cells (Mann et al., Cell 33:153-159 (1983); FLY (Cossett et al., Virol. 193:385-395 (1993); BOSC 23 cells (Pear et al., PNAS 90:8392-8396 (1993); PA317 cells (Miller et al., Molec. and Cell. Biol. 6:2895-2902 (1986); Kat cell line (Finer et al., Blood 83:43-50 (1994)); GP+E cells and GP+EM12 cells (Markowitz et al., J. Virol. 62:1120-1124 (1988), and Psi Crip and Psi Cre cells (U.S. Pat. No. 5,449,614; Mulligan et al., PNAS 85:6460-6464 (1988)).

Packaging cells useful for the production of recombinant viral particles also include insect cells. Any insect cell that allows for replication of a virus (*e.g*., lentivirus or AAV) and that can be maintained in culture can be used in the methods described herein. Nonlimiting, exemplary insect cells that can be used as packaging cells include cells from *Spodoptera frugiperda,* drosophila, or mosquito (*e.g., Aedes albopictus*)*.* Particular insect cells are cells from insect species that are susceptible to baculovirus infection, including, without limitation, Se301, SeIZD2109, SeUCR1, Sf9, Sf900+, Sf21, BTI-TN-5B1-4, MG-1, Tn368, HzAm1, Ha2302, Hz2E5, and High Five™ cells (Invitrogen).

Packaging cells can be transfected with a vector containing a gene of interest and a packaging signal, as well as one or more exogenous mRNA molecules described herein. Any known cell transfection technique can be used. Generally cells are incubated (*i.e.,* cultured) with the vector in an appropriate medium under suitable transfection conditions, as is well known in the art. For example, methods such as electroporation, lipofection, polyethylenimine (PEI), and calcium phosphate precipitation can be used.

Positive packaging cell transformants (*i.e.,* cells which have taken up and integrated the vector containing a gene of interest and/or an mRNA) can be identified using a variety of selection markers known in the art. For example, marker genes, such as green fluorescent protein (GFP), hygromycin resistance (Hyg), neomycin resistance (Neo) and beta-galactosidase (beta-gal) genes can be included in the vector and assayed using, *e.g*., enzymatic activity or drug resistance assays. Alternatively, cells can be assayed for reverse transcriptase (RT) activity as described by Goff et al., J. Virol. 38:239 (1981) as a measure of viral protein production.

Similar assays can be used to test for the production by packaging cells of unwanted, replication-competent helper virus. For example, marker genes, such as those described above, can be included in the vector containing the viral packaging sequence (ψ) and LTRs. Following transient transfection of packaging cells with the vector, packaging cells can be subcultured with other non-packaging cells. These non-packaging cells will be infected with recombinant, replication-deficient retroviral vectors of the invention carrying the marker gene. However, because these non-packaging cells do not contain the genes necessary to produce viral particles (*e.g.,* gag, pol and env genes), they should not, in turn, be able to infect other cells when subcultured with these other cells. If these other cells are positive for the presence of the marker gene when subcultured with the non-packaging cells, then unwanted, replication-competent virus has been produced.

Accordingly, to test for the production of unwanted helper-virus, packaging cells of the invention can be subcultured with a first cell line (*e.g.,* NIH3T3 cells) that in turn is subcultured with a second cell line that is tested for the presence of a marker gene or RT activity, indicating the presence of replication-competent helper retrovirus. Marker genes can be assayed using *e.g.,* FACS, staining and enzymatic activity assays, as is well known in the art.

### Purification/Isolation

Recombinant viral particles can be purified from packaging cells using known methods. General methods include centrifugation or ultracentrifugation with CsC1 gradient or sucrose gradient, or iodixanol gradient, filtration (*e.g*., using a 0.22 µm filter), chromatography or combinations thereof (see, *e.g.,* Duffy et al., Gene Therapy 12:S62-S72 (2005); Tiscornia et al., Nat. Protoc. 1:241-245 (2006); Gao et al., Hum. Gene Ther. 11:2079-2091 (2000)).

If the packaging cells are cultured in a suspension culture, recombinant viruses can be harvested from the supernatant of the culture medium.

Recombinant viral particles can also be purified by affinity-purification using an anti-viral antibodies, *e.g*., immobilized antibodies. The anti-viral antibody can be a monoclonal or polyclonal antibody, *e.g*., an antibody recognizing a capsid protein. Particular antibodies that can be used for purification are single chain camelid antibodies or a fragment thereof (see, *e.g.,* Muyldermans, Biotechnol. 74:277-302 (2001)).

Thus, in some embodiments, the present invention provides a population of purified recombinant viral particles produced using inventive methods described herein. It is contemplated that purified recombinant viral particles according to the present invention have improved ratio of full to empty capsids/virions. In some embodiments, the amount of full capsids/virions constitutes more than about 0.25%, 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or 100% of the total population of purified recombinant viral particles. In some embodiments, the amount of empty capsids/virions constitutes less than about 99%, 98%, 97%, 96%, 95%, 90%, 85%, 80%, 75%, 70%, 65%, 60%, 55%, 50%, 45%, 40%, 35%, 30%, 25%, 20%, 15%, 10%, 5%, 4%, 3%, 2%, or 1% of the total population of purified recombinant viral particles. In some embodiments, the ratio of full to empty capsids/virions in a population of purified recombinant viral particles is greater than about 1:5000, 1:4000, 1:3000, 1:2000, 1:1000, 1:500, 1:400, 1:300, 1:200, 1:100, 1:50, 1:40, 1:30, 1:25, 1:20, 1:10, 1:8, 1:6, 1:5, 1:4, 1:3, 1:2, 1:1,2:1,3:1,4:1,5:1,6:1,8:1, 10:1,20:1,25:1,30:1,40:1,50:1, 100:1,200:1, 500:1, or 1000:1.

### Pharmaceutical Compositions and Administration

Recombinant viral systems are commonly used as delivery systems for, *inter alia,* the transfer of a gene of interest to one or more target cells. The transfer can occur *in vitro, ex vivo, in vivo,* or combinations thereof. A recombinant viral particle is capable of transducing a recipient cell with a gene of interest. Once within the cell, a DNA or RNA genome from the viral particle can be integrated into the genome of the recipient cell (with or without reverse transcription) or present as an extra-chromosomal construct such as plasmid.

In particular embodiments, the present invention provides a pharmaceutical composition comprising a recombinant viral particle described herein and a pharmaceutically acceptable carrier, excipient, or diluent. For injection, the carrier can be, *e.g.,* a liquid. For other methods of administration, the carrier can be, *e.g.,* a solid or liquid. For inhalation administration, the carrier can be respirable, and optionally can be in solid or liquid particulate form. Acceptable carriers or diluents are well known in the art, and are described in, *e.g.,* Remington's Pharmaceutical Sciences, Mack Publishing Co. (A. R. Gennaro ed. 1985). The choice of pharmaceutical carrier, excipient or diluent can be selected with regard to the intended route of administration and standard pharmaceutical practice. The pharmaceutical compositions may comprise as--or in addition to--the carrier, excipient or diluent any suitable binder(s), lubricant(s), suspending agent(s), coating agent(s), solubilizing agent(s).

Preservatives, stabilizers, dyes and even flavouring agents can be provided in the pharmaceutical composition. Examples of preservatives include sodium benzoate, sorbic acid and esters of p-hydroxybenzoic acid. Antioxidants and suspending agents can also be used.

Different composition/formulation requirements can be determined by different delivery systems. By way of example, the pharmaceutical composition of the present invention can be formulated to be delivered using a mini-pump or by a mucosal route, for example, as a nasal spray or aerosol for inhalation or ingestible solution, or parenterally in which the composition is formulated by an injectable form, for delivery, by, for example, an intravenous, intramuscular or subcutaneous route. Alternatively, the formulation can be designed to be delivered by both routes.

Where the pharmaceutical composition is to be delivered mucosally through the gastrointestinal mucosa, it should be able to remain stable during transit though the gastrointestinal tract; for example, it should be resistant to proteolytic degradation, stable at acid pH and resistant to the detergent effects of bile. Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose or chalk, or in capsules or ovules either alone or in admixture with excipients, or in the form of elixirs, solutions or suspensions containing flavouring or colouring agents, or they can be injected parenterally, for example, intravenously, intramuscularly or subcutaneously. For parenteral administration, the compositions can be in the form of a sterile aqueous solution, which can contain other substances, for example, enough salts or monosaccharides to make the solution isotonic with blood. For buccal or sublingual administration, the compositions can be administered in the form of tablets or lozenges that can be formulated in a conventional manner.

By "pharmaceutically acceptable" it is meant a material that is not toxic or otherwise undesirable, *i.e.,* the material may be administered to a subject without causing any or an acceptable level of undesirable biological effects.

In some instances, a recombinant viral particle described herein can be transduced into a cell, *e.g., in vivo, in vitro* or *ex vivo.* For example, if the cell is a cell from a mammalian subject, the cell can be removed from the subject, transduced by the viral particle, and prepared for reimplantation into the subject (*ex vivo* transduction). Alternatively, the cell can be transduced by direct gene transfer *in vivo* by administering a recombinant viral particle to a subject in accordance with standard techniques. Further, the cell can be a stable cell line and can be transduced *in vitro* using standard techniques.

The cell(s) into which the virus particle is introduced can be of any type, including but not limited to neural cells (including cells of the peripheral and central nervous systems, in particular, brain cells such as neurons and oligodendricytes), lung cells, cells of the eye (including retinal cells, retinal pigment epithelium, and corneal cells), epithelial cells (*e.g.,* gut and respiratory epithelial cells), muscle cells (*e.g.,* skeletal muscle cells, cardiac muscle cells, smooth muscle cells and/or diaphragm muscle cells), dendritic cells, pancreatic cells (including islet cells), hepatic cells, myocardial cells, bone cells (*e.g.,* bone marrow stem cells), hematopoietic stem cells, spleen cells, keratinocytes, fibroblasts, endothelial cells, prostate cells, germ cells, and the like. In some instances, the cell can be any progenitor cell. As a further possibility, the cell can be a stem cell (*e.g.,* neural stem cell, liver stem cell). As still a further alternative, the cell can be a cancer or tumor cell.

When transferring a nucleic acid to a cell *in vitro,* a viral particle can be introduced into the cell at an appropriate multiplicity of infection according to standard transduction methods suitable for the particular target cells. Titers of virus vector for administration can vary, depending upon the target cell type, number of target cells, and the particular virus vector, and can be determined by those of skill in the art without undue experimentation. For example, at least about 10³ infectious units, or at least about 10⁵ infectious units are introduced to the cell.

The viral particle can be introduced into cells *in vitro,* and the transduced cell can subsequently be administered to a subject. In particular embodiments, the cells are harvested from a subject, the virus vector is introduced therein, and the cells are then administered back into the subject. Methods of harvesting cells from a subject for manipulation *ex vivo,* followed by introduction back into the subject, are known in the art (see, *e.g.,* U.S. Pat. No. 5,399,346). In other methods, the recombinant virus vector is introduced into cells from a donor subject, into cultured cells, or into cells from any other suitable source, and the cells are administered to a subject.

Dosages of transduced cells (*e.g*., transduced with a viral particle described herein) for administration to a subject can vary upon the age, condition, and species of the subject, the type of cell, the nucleic acid being expressed by the cell, the mode of administration, and the like. In exemplary methods, at least about 10² to about 10⁸ cells, or at least about 10³ to about 10⁶ cells are administered per dose in a pharmaceutically acceptable carrier. In particular embodiments, the cells transduced with the viral particles are administered to the subject in an effective amount in combination with a pharmaceutical carrier.

In some embodiments, a viral particle is introduced into a cell, and the cell is administered to a subject to elicit an immunogenic response against a delivered polypeptide (*e.g*., expressed as a transgene or in the capsid). A dosage of cells expressing an immunogenically effective amount of the polypeptide in combination with a pharmaceutically acceptable carrier is administered. An "immunogenically effective amount", as used herein, is an amount of the expressed polypeptide that is sufficient to evoke an active immune response against the polypeptide in the subject to which the pharmaceutical formulation is administered. In particular embodiments, the dosage is sufficient to produce a protective immune response.

In other instances, a viral particle is administered to a subject *in vivo.* Administration of the viral particle to a human subject or an animal in need thereof can be by any means known in the art. The viral particle can be delivered in an effective dose in a pharmaceutically acceptable carrier.

The viral particles described herein can be administered to a subject to elicit an immunogenic response (e.g., as a vaccine). An immunogenic composition can include an immunogenically effective amount of viral particles in combination with a pharmaceutically acceptable carrier, such as to produce a protective immune response.

Dosages of the viral particles to be administered to a subject can depend upon the mode of administration, the disease or condition to be treated and/or prevented, the individual subject's condition, the particular viral particle, the nucleic acid to be delivered, and the like, and can be determined in a routine manner. Exemplary doses for achieving therapeutic effects are titers of at least about 10⁵, 10⁶, 10⁷, 10⁸, 10⁹, 10¹⁰, 10¹¹, 10¹², 10¹³, 10¹⁴, or 10¹⁵ transducing units, or about 10⁸-10¹³ transducing units.

In particular instances, more than one administration (*e.g*., two, three, four or more administrations) are used to achieve a desired level of gene expression over a period of various intervals, *e.g.,* daily, weekly, monthly, yearly, *etc.*

Exemplary modes of administration include oral, rectal, transmucosal, intranasal, inhalation (*e.g.,* via an aerosol), buccal (*e.g.,* sublingual), vaginal, intrathecal, intraocular, transdermal, in utero (or in ovo), parenteral (*e.g*., intravenous, subcutaneous, intradermal, intramuscular, intradermal, intrapleural, intracerebral, and intraarticular), topical *(e.g.,* to both skin and mucosal surfaces, including airway surfaces, and transdermal administration), intralymphatic, and the like, as well as direct tissue or organ injection (*e.g*., to liver, skeletal muscle, cardiac muscle, diaphragm muscle or brain). Administration can also be to a tumor (*e.g.,* in or near a tumor or a lymph node). The route of administration in any given case can depend on the nature and severity of the condition being treated and/or prevented and on the nature of the particular vector that is being used.

Injectable formulations of a viral particle described herein can be prepared in conventional forms, either as liquid solutions or suspensions, solid forms suitable for solution or suspension in liquid prior to injection, or as emulsions. Alternatively, a viral particle described herein can be administered locally, rather than systemically, for example, in a depot or sustained-release formulation. Further, a viral particle can be delivered adhered to a surgically implantable matrix (*e.g*., as described in U.S. Patent Publication No. US 20040013645).

The viral particles described herein can be administered to the lungs of a subject by any suitable means, optionally by administering an aerosol suspension of respirable particles comprised of the viral particles, which the subject inhales. The respirable particles can be liquid or solid. Aerosols of liquid particles comprising the viral particles can be produced by any suitable means, such as with a pressure-driven aerosol nebulizer or an ultrasonic nebulizer, as is known to those of skill in the art (see, *e.g.,* U.S. Pat. No. 4,501,729). Aerosols of solid particles comprising the viral particles can also be produced with any solid particulate medicament aerosol generator, by techniques known in the pharmaceutical art.

In some instances, a subject is screened for one or more neutralizing antibodies to a particular virus or serotype (see, e.g., Li et al., Gene Therapy 19:288-294 (2012)). For example, in some embodiments, prior to administering a recombinant viral particle described herein to a subject, a biological sample (e.g., blood) is obtained from the subject and the presence or absence of one or more antibody (e.g., neutralizing antibody) to one or more virus or serotypes is determined. If the presence of an antibody (e.g., a neutralizing antibody) to a particular virus or serotype is identified, a viral particle described herein but derived from a different virus or serotype (e.g., one against which few or no antibodies is present) is administered to the subject.

### Diseases and Therapeutic Polypeptides

Recombinant viral particles produced using methods described herein can be used to delivery nucleic acids to cells that encode therapeutic (*e.g*., for medical or veterinary uses) or immunogenic (*e.g*., for vaccines) polypeptides. In particular instances, a viral particle is used to deliver nucleic acids that encode a therapeutic polypeptide (*e.g*., replacement enzyme) for the treatment of a lysosomal storage disease.

### Lysosomal Storage Diseases and Replacement Enzymes

Any lysosomal storage disease can be treated using a viral particle described herein, in particular those lysosomal storage diseases having CNS etiology and/or symptoms, including, but not limited to, aspartylglucosaminuria, cholesterol ester storage disease, Wolman disease, cystinosis, Danon disease, Fabry disease, Farber lipogranulomatosis, Farber disease, fucosidosis, galactosialidosis types I/II, Gaucher disease types I/II/III, globoid cell leukodystrophy, Krabbe disease, glycogen storage disease II, Pompe disease, GM1- gangliosidosis types I/II/III, GM2-gangliosidosis type I, Tay Sachs disease, GM2-gangliosidosis type II, Sandhoff disease, GM2-gangliosidosis, a-mannosidosis types I/II, beta-mannosidosis, metachromatic leukodystrophy, mucolipidosis type I, sialidosis types I/II, mucolipidosis types II/III, I-cell disease, mucolipidosis type IIIC pseudo-Hurler polydystrophy, mucopolysaccharidosis type I, mucopolysaccharidosis type II, Hunter syndrome, mucopolysaccharidosis type IIIA, Sanfilippo syndrome (type A, B, C or D), mucopolysaccharidosis type IIIB, mucopolysaccharidosis type IIIC, mucopolysaccharidosis type IIID, mucopolysaccharidosis type IVA, Morquio syndrome, mucopolysaccharidosis type IVB, mucopolysaccharidosis type VI, mucopolysaccharidosis type VII, Sly syndrome, mucopolysaccharidosis type IX, multiple sulfatase deficiency, neuronal ceroid lipofuscinosis, CLN1 Batten disease, CLN2 Batten diseae, Niemann-Pick disease types A/B, Niemann-Pick disease type C1, Niemann-Pick disease type C2, pycnodysostosis, Schindler disease types I/II, Gaucher disease and sialic acid storage disease.

In some embodiments, lysosomal storage diseases to be treated using viral particles described herein include Hunters Syndrome, metachromatic leukodystrophy (MLD) disease, Sanfilippo syndrome type A, Sanfilippo syndrome type B, and globoid cell leukodystrophy (GLD) disease.

A detailed review of the genetic etiology, clinical manifestations, and molecular biology of the lysosomal storage diseases are detailed in Scriver et al., eds., The Metabolic and Molecular Bases of Inherited Disease, 8th Ed., Vol. II, McGraw Hill, (2001). Thus, the enzymes deficient in the above diseases are known to those of skill in the art, some of these are exemplified in the Table below:

**Table 1:**

| Disease Name | Enzyme **Deficiency** | Substance |
|---|---|---|
| | | Stored |
| Pompe Disease | Acid-a1, 4-Glucosidase | Glycogen α□1-4 linked Oligosaccharides |
| GM1 Gangliodsidosis | β-Galactosidase | GM₁ Gangliosides |
| Tay-Sachs Disease | β-Hexosaminidase A | GM₂ Ganglioside |
| GM2 Gangliosidosis: AB Variant | GM₂ Activator Protein | GM₂ Ganglioside |
| Sandhoff Disease | β-Hexosaminidase A&B | GM₂ Ganglioside |
| Fabry Disease | α-Galactosidase A | Globosides |
| Gaucher Disease | Glucocerebrosidase | Glucosylceramide |
| Metachromatic Leukodystrophy | Arylsulfatase A | Sulphatides |
| Krabbe Disease | Galactosylceramidase | Galactocerebroside |
| Niemann Pick, Types A & B | Acid Sphingomyelinase | Sphingomyelin |
| Niemann-Pick, Type C | Cholesterol Esterification Defect | Sphingomyelin |
| Niemann-Pick, Type D | Unknown | Sphingomyelin |
| Farber Disease | Acid Ceramidase | Ceramide |
| Wolman Disease | Acid Lipase | Cholesteryl Esters |
| Hurler Syndrome | α-L-Iduronidase | Heparan & Dermatan Sulfates |
| (MPS IH) | | |
| Scheie Syndrome | α-L-Iduronidase | Heparan & Dermatan, Sulfates |
| (MPS IS) | | |
| Hurler-Scheie | α-L-Iduronidase | Heparan & Dermatan Sulfates |
| (MPS IH/S) | | |
| Hunter Syndrome | Iduronate Sulfatase | Heparan & Dermatan Sulfates |
| (MPS II) | | |
| Sanfilippo syndrome type A | Heparan N-Sulfatase | Heparan Sulfate |
| (MPS IIIA) | | |
| Sanfilippo syndrome type B | α-N-Acetylglucosaminidase | Heparan Sulfate |
| (MPS IIIB) | | |
| Sanfilippo syndrome | Acetyl-CoA-Glucosaminide Acetyltransferase | Heparan |
| type C | | Sulfate |
| (MPS IIIC) | | |
| Sanfilippo syndrome | N-Acetylglucosamine-6-Sulfatase | Heparan |
| type D | | Sulfate |
| (MPS IIID) | | |
| Morquio B | β-Galactosidase | Keratan |
| (MPS IVB) | | Sulfate |
| Maroteaux-Lamy | Arylsulfatase B | Dermatan |
| (MPS VI) | | Sulfate |
| Sly Syndrome | β-Glucuronidase | |
| (MPS VII) | | |
| α-Mannosidosis | α-□Mannosidase | Mannose/Oligosaccharides |
| β -Mannosidosis | β-Mannosidase | Mannose/Oligosaccharides |
| Fucosidosis | α-L-Fucosidase | Fucosyl Oligosaccharides |
| Aspartylglucosaminuria | N-Aspartyl-β -Glucosaminidase | Aspartylglucosamine Asparagines |
| Sialidosis (Mucolipidosis I) | α-Neuraminidase | Sialyloligosaccharides |
| Galactosialidosis (Goldberg Syndrome) | Lysosomal Protective Protein Deficiency | Sialyloligosaccharides |
| Schindler Disease | α-N-Acetyl-Galactosaminidase | |
| Mucolipidosis II (I-Cell Disease) | N-Acetylglucosamine-1-Phosphotransferase | Heparan Sulfate |
| Mucolipidosis III (Pseudo-Hurler Polydystrophy) | Same as ML II | |
| Cystinosis | Cystine Transport Protein | Free Cystine |
| Salla Disease | Sialic Acid Transport Protein | Free Sialic Acid and Glucuronic Acid |
| Infantile Sialic Acid Storage Disease | Sialic Acid Transport Protein | Free Sialic Acid and Glucuronic Acid |
| Infantile Neuronal Ceroid Lipofuscinosis | Palmitoyl-Protein Thioesterase | Lipofuscins |
| Mucolipidosis IV | Unknown | Gangliosides & Hyaluronic Acid |
| Prosaposin | Saposins A, B, C or D | |

The viral particles described herein can be used to deliver any replacement enzyme. As used herein, replacement enzymes include, *e.g*., any enzyme that can act to replace at least partial activity of the deficient or missing lysosomal enzyme in a lysosomal storage disease to be treated. In some embodiments, a replacement enzyme is capable of reducing accumulated substance in lysosomes or is capable of rescuing or ameliorating one or more lysosomal storage disease symptoms.

In some embodiments, a suitable replacement enzyme may be any lysosomal enzyme known to be associated with the lysosomal storage disease to be treated. In some embodiments, a suitable replacement enzyme is an enzyme selected from the enzyme listed in Table 1 above. In some embodiments, a replacement enzyme suitable is iduronate-2-sulfatase (I2S), arylsulfatase A (ASA), heparan N-sulfatase (HNS), alpha-N-acetylglucosaminidase (Naglu) or β-galactosidase (GLC).

Additional, nonlimiting examples of diseases that can be treated using recombinant viral particles described herein include Huntington's disease (where the gene of interest is, e.g., HTT), Parkinson's disease, Batten disease (ceroid lipofuscinosis, all forms), spinal muscular atrophy (where the gene of interest is, e.g., SMN1), X-linked adrenoleukodystrophy (where the gene of interest is, e.g., ABCD1), muscular dystrophies (Duchenne, Becker, etc), Hemophilia B (where the gene of interest is, e.g., FIX), Hemophilia A (where the gene of interest is, e.g., FVIII), Hereditary hemorrhagic telangiectasia (where the gene of interest is, e.g., endoglin), Alport syndrome (where the gene of interest is, e.g., COL4A5), Fragile X (where the gene of interest is, e.g., FMR1), X-linked agammaglobulinemia (where the gene of interest is, e.g., BTK), Urea Cycle Diseases, glycogen storage diseases, and Cystic fibrosis (where the gene of interest is, e.g., CFTR).

### INCORPORATION-BY-REFERENCE

All publications, patent applications, patents, and other references mentioned herein are incorporated by reference in their entirety. In addition, the materials, methods, and examples are illustrative only and not intended to be limiting. Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described herein.

### EQUIVALENTS

It is to be understood that while the disclosure has been described in conjunction with the detailed description thereof, the foregoing description is intended to illustrate and not limit the scope of the invention, which is defined by the scope of the appended claims. Other aspects, advantages, and modifications are within the scope of the following claims.

The invention provides the following numbered embodiments:
1. A method of producing a lentiviral particle, the method comprising:
   introducing into a packaging cell one or more exogenous mRNAs encoding one or more helper factors for assembling transduction-competent lentiviral particles, wherein the packaging cell comprises a gene of interest; and
   culturing the packaging cell under conditions suitable for the packaging cell to produce a lentiviral particle comprising the gene of interest.
2. The method of embodiment 1, wherein the one or more exogenous mRNAs are *in vitro* transcribed mRNAs or synthetic mRNAs.
3. The method of embodiment 2, wherein the exogenous mRNAs are stabilized mRNAs.
4. The method of embodiment 1, wherein the one or more helper factors are selected from the group consisting of a Pol protein, a Gag protein, an Env protein, and a combination thereof.
5. The method of embodiment 4, wherein the one or more helper factors comprise a Pol protein, a Gag protein, and an Env protein.
6. The method of any one of the preceding embodiments, wherein the one or more helper factors are encoded by one single exogenous transcribed mRNA molecule.
7. The method of any one of embodiments 1-5, wherein the one or more helper factors are encoded by two or more exogenous mRNA molecules.
8. The method of embodiment 7, wherein each of the one or more helper factors is encoded by a separate exogenous mRNA molecules.
9. The method of any one of the preceding embodiments, wherein the one or more helper factors further comprise a lentiviral Tat protein and/or a lentiviral Rev protein.
10. The method of any one of the preceding embodiments, wherein the one or more exogenous mRNAs are introduced by electroploration, lipofection, PEI, or combination thereof.
11. The method of any one of the preceding embodiments, wherein the gene of interest is integrated in the genome of the packaging cell.
12. The method of any one of embodiments 1-10, wherein the gene of interest is present on a extra-chromosomal plasmid.
13. The method of embodiment 12, wherein the gene of interest is transiently transfected into the packaging cell.
14. The method of any one of the preceding embodiments, wherein the gene of interest is associated with a packaging signal.
15. The method of any one of the preceding embodiments, wherein the gene of interest is operably linked to a promoter.
16. The method of any one of the preceding embodiments, wherein the gene of interest is framed by a left and a right long terminal repeat (LTR) sequence.
17. The method of any one of the preceding embodiments, wherein the packaging cell is a mammalian cell.
18. The method of embodiment 17, wherein the mammalian cell is a human cell.
19. The method of embodiment 18, wherein the human cell is a HEK293 cell.
20. The method of embodiment 17, wherein the mammalian cell is a CHO cell.
21. The method of any one of the preceding embodiments, wherein the method further comprises a step of purifying the lentiviral particles.
22. A method of producing a lentiviral particle, the method comprising
   introducing into a packaging cell (i) an exogenous mRNA encoding a lentiviral Gag protein, (ii) an exogenous mRNA encoding a lentiviral Pol protein, and (iii) an exogenous mRNA encoding a lentiviral Env protein; wherein the packaging cell comprises a gene of interest associated with a packaging signal; and
   culturing the packaging cell under conditions suitable for the packaging cell to produce a lentiviral particle comprising the gene of interest.
23. The method of embodiment 22, wherein the exogenous mRNAs are *in vitro* transcribed mRNAs or synthetic mRNAs.
24. The method of embodiment 23, wherein the exogenous mRNAs are stabilized mRNAs.
25. A population of lentiviral particles produced using a method according to any one of the preceding embodiments.
26. A packaging cell capable of producing a lentiviral particle, comprising:
   one or more exogenous mRNAs encoding one or more helper factors for assembling transduction-competent lentiviral particles, wherein the packaging cell comprises a gene of interest associated with a packaging signal.
27. The packaging cell of embodiment 26, wherein the one or more exogenous mRNAs are *in vitro* transcribed mRNAs or synthetic mRNAs.
28. The method of embodiment 27, wherein the one or more exogenous mRNAs are stabilized mRNAs.
29. The packaging cell of embodiment 26, wherein the one or more helper factors are selected from the group consisting of a lentiviral Gag protein, Pol protein, Env protein, and combination thereof.
30. The packaging cell of embodiment 29, wherein the one or more helper factors further comprise a lentiviral Tat protein and/or a lentiviral Rev protein.
31. The packaging cell of any one of embodiments 26-30, wherein the gene of interest is integrated in the genome of the packaging cell.
32. The packaging cell of any one of embodiments 26-30, wherein the gene of interest is present as an extra-chromosomal plasmid vector.
33. The packaging cell of any one of embodiments 26-32, wherein the packaging cell is a mammalian cell.
34. The packaging cell of embodiment 33, wherein the mammalian cell is a human cell.
35. The packaging cell of embodiment 34, wherein the human cell is an HEK293 cell.
36. A system for producing a lentiviral particle, comprising
   one or more constructs encoding one or more helper factors for assembling transduction-competent lentiviral particles;
   reagents for *in vitro* transcription of mRNAs from the one or more constructs encoding one or more helper factors;
   a packaging cell; and
   reagents for introducing the *in vitro* transcribed mRNAs into the packaging cell.
37. The system of embodiment 36, wherein the packaging cell comprises a gene of interest.
38. A method of producing an adeno-associated virus (AAV) particle, the method comprising:
   introducing into a packaging cell one or more exogenous mRNAs encoding one or more helper factors for assembling transduction-competent AAV particles, wherein the packaging cell comprises a gene of interest; and
   culturing the packaging cell under conditions suitable for the packaging cell to produce an AAV particle comprising the gene of interest.
39. The method of embodiment 38, wherein the one or more exogenous mRNAs are *in vitro* transcribed mRNAs or synthetic mRNAs.
40. The method of embodiment 39, wherein the one or more exogenous mRNAs are stabilized mRNAs.
41. The method of embodiment 38, wherein the one or more helper factors are selected from the group consisting of a Rep78 protein, a Rep 52 protein, an AAP protein, a capsid protein, and combination thereof.
42. The method of embodiment 41, wherein the one or more helper factors comprise a Rep78 protein, a Rep 52 protein, an AAP protein, and a capsid protein.
43. The method of any one of embodiments 38-42, wherein the one or more helper factors are encoded by one single exogenous mRNA molecule.
44. The method of any one of embodiments 38-42, wherein the one or more helper factors are encoded by two or more exogenous mRNA molecules.
45. The method of embodiment 44, wherein each of the one or more helper factors is encoded by a separate exogenous mRNA molecule.
46. The method of any one of embodiments 38-45, wherein the one or more exogenous mRNAs are introduced by electroporation, lipofection, PEI, or combination thereof.
47. The method of any one of embodiments 38-46, wherein the gene of interest is integrated in the genome of the packaging cell.
48. The method of any one of embodiments 38-46, wherein the gene of interest is present on a extra-chromosomal plasmid.
49. The method of embodiment 48, wherein the gene of interest is transiently transfected into the packaging cell.
50. The method of any one of embodiments 38-49, wherein the gene of interest is associated with a packaging signal.
51. The method of any one of embodiments 38-50, wherein the gene of interest is operably linked to a promoter.
52. The method of any one of embodiments 38-51, wherein the gene of interest is framed by a left and a right inverted terminal repeat (ITR) sequence.
53. The method of any one of embodiments 38-52, wherein the AAV particle is an AAV1, AAV2, AAV5, AAV6, or AAV8 particle.
54. The method of any one of embodiments 38-53, wherein the packaging cell is a mammalian cell.
55. The method of embodiment 54, wherein the mammalian cell is a human cell.
56. The method of embodiment 55, wherein the human cell is a HEK293 cell.
57. The method of any one of embodiments 38-53, wherein the packaging cell is an insect cell.
58. The method of embodiment 57, wherein the insect cell is an SF9 cell.
59. The method of any one of embodiments 38-58, wherein the method further comprises a step of purifying the AAV particles.
60. A method of producing an AAV particle, the method comprising
   introducing into a packaging cell (i) an exogenous mRNA encoding a Rep 78 protein, (ii) an exogenous mRNA encoding a Rep 52 protein, and (iii) an exogenous mRNA encoding a capsid protein; wherein the packaging cell comprises a gene of interest associated with a packaging signal; and
   culturing the packaging cell under conditions suitable for the packaging cell to produce an AAV particle comprising the gene of interest.
61. The method of embodiment 60, wherein the exogenous mRNAs are *in vitro* transcribed mRNAs or synthetic mRNAs.
62. The method of embodiment 61, wherein the exogenous mRNAs are stabilized mRNAs.
63. A population of AAV particles produced using a method according to any one of embodiments 38-60.
64. A packaging cell capable of producing an AAV particle, comprising:
   one or more exogenous mRNAs encoding one or more helper factors for assembling transduction-competent AAV particles, wherein the packaging cell comprises a gene of interest associated with a packaging signal.
65. The packaging cell of embodiment 64, wherein the one or more exogenous mRNAs are *in vitro* transcribed mRNAs or synthetic mRNAs.
66. The packaging cell of embodiment 65, wherein the one or more exogenous mRNAs are stabilized mRNAs.
67. The packaging cell of embodiment 64, wherein the one or more helper factors are selected from the group consisting of a Rep 78 protein, a Rep 52 protein, an AAP protein, a capsid protein, and a combination thereof.
68. The packaging cell of any one of embodiments 64-67, wherein the gene of interest is integrated in the genome of the packaging cell.
69. The packaging cell of any one of embodiments 64-67, wherein the gene of interest is present on an extra-chromosomal plasmid vector.
70. The packaging cell of embodiment 69, wherein the gene of interest is transiently transfected into the packaging cell.
71. The packaging cell of any one of embodiments 64-70, wherein the packaging cell is a mammalian cell.
72. The packaging cell of embodiment 71, wherein the mammalian cell is a human cell.
73. The packaging cell of embodiment 72, wherein the human cell is an HEK293 cell.
74. The packaging cell of any one of embodiments 64-70, wherein the packaging cell is an insect cell.
75. The packaging cell of embodiment 74, wherein the insect cell is an SF9 cell.
76. A system for producing an AAV particle, comprising
   one or more constructs encoding one or more helper factors for assembling transduction-competent AAV particles;
   reagents for *in vitro* transcription of mRNAs from the one or more constructs encoding one or more helper factors;
   a packaging cell; and
   reagents for introducing the *in vitro* transcribed mRNAs into the packaging cell.
77. The system of embodiment 76, wherein the packaging cell comprises a gene of interest.
78. A method of treating a subject having or at risk of a lysosomal storage disease, the method comprising administering to the subject an effective amount of a lentiviral particle produced by the method of any one of embodiments 1-24, wherein the gene of interest encodes a lysosomal enzyme.
79. A method of treating a subject having or at risk of a lysosomal storage disease, the method comprising administering to the subject an effective amount of an AAV particle produced by the method of any one of embodiments 38-62, wherein the gene of interest encodes a lysosomal enzyme.
80. A method of producing a viral particle, the method comprising:
   introducing into a packaging cell one or more exogenous mRNAs encoding one or more helper factors for assembling transduction-competent viral particles, wherein the one or more exogenous mRNAs are not self-replicating mRNAs, and wherein the packaging cell comprises a gene of interest; and
   culturing the packaging cell under conditions suitable for the packaging cell to produce a viral particle comprising the gene of interest.
81. The method of embodiment 80, wherein the one or more exogenous mRNAs do not comprise alpha virus sequence.
82. The method of embodiment 80, wherein the one or more exogenous mRNAs do not comprise Semliki Forest virus (SFV) sequence.
83. The method of embodiment 80, wherein the viral particle is a lentiviral particle.
84. The method of embodiment 80, wherein the viral particle is an AAV particle.
85. A packaging cell capable of producing a viral particle, comprising:
   one or more exogenous mRNAs encoding one or more helper factors for assembling transduction-competent viral particles, wherein the one or more exogenous mRNAs are not self-replicating mRNAs, and wherein the packaging cell comprises a gene of interest associated with a packaging signal.
86. The packaging cell of embodiment 85, wherein the one or more exogenous mRNAs do not comprise alpha virus sequence.
87. The packaging cell of embodiment 85, wherein the one or more exogenous mRNAs do not comprise Semliki Forest virus (SFV) sequence.
88. The packaging cell of embodiment 85, wherein the viral particle is a lentiviral particle.
89. The packaging cell of embodiment 85, wherein the viral particle is an AAV particle.

## Claims

1. A method of producing a viral particle, the method comprising:
introducing into a packaging cell one or more exogenous mRNAs encoding one or more helper factors for assembling transduction-competent viral particles, wherein the one or more exogenous mRNAs are not self-replicating mRNAs and optionally do not comprise alpha virus sequence or Semliki Forest virus (SFV) sequence, and wherein the packaging cell comprises a gene of interest; and
culturing the packaging cell under conditions suitable for the packaging cell to produce a viral particle comprising the gene of interest.

2. A packaging cell capable of producing a viral particle, comprising:
one or more exogenous mRNAs encoding one or more helper factors for assembling transduction-competent viral particles, wherein the one or more exogenous mRNAs are not self-replicating mRNAs and optionally do not comprise alpha virus sequence or Semliki Forest virus (SFV) sequence, and wherein the packaging cell comprises a gene of interest associated with a packaging signal.

3. The method of claim 1 or the packaging cell of claim 2, wherein the one or more exogenous mRNAs are *in vitro* transcribed mRNAs or synthetic mRNAs, optionally wherein the exogenous mRNAs are stabilized mRNAs.

4. The method or packaging cell of any one of the preceding claims, wherein (a) the one or more helper factors are encoded (i) by one single exogenous transcribed mRNA molecule or (ii) by two or more exogenous mRNA molecules, optionally wherein each of the one or more helper factors is encoded by a separate exogenous mRNA molecules.

5. The method of any one of the preceding claims, wherein the one or more exogenous mRNAs are introduced by electroploration, lipofection, PEI, or combination thereof.

6. The method or packaging cell of any one of the preceding claims, wherein
(a) the gene of interest is (i) integrated in the genome of the packaging cell or (ii) present on an extra-chromosomal plasmid, optionally wherein the gene of interest is transiently transfected into the packaging cell, and/or
(b) the gene of interest is associated with a packaging signal, and/or
(c) the gene of interest is operably linked to a promoter, and/or
(e) the packaging cell is a mammalian cell, optionally wherein the mammalian cell is (i) a human cell, optionally a HEK293 cell, (ii) a CHO cell, or (iii) an insect cell, optionally an SF9 cell.

7. The method of any one of the preceding claims, wherein the method further comprises a step of purifying the viral particles.

8. The method or the packaging cell of any one of the preceding claims, wherein the viral particle is a lentiviral particle.

9. The method or packaging cell of claim 8, wherein the gene of interest is framed by a left and a right long terminal repeat (LTR) sequence.

10. The method or packaging cell of claim 8 or 9, wherein the one or more helper factors are selected from the group consisting of a Pol protein, a Gag protein, an Env protein, and a combination thereof, optionally wherein the one or more helper factors (i) comprise a Pol protein, a Gag protein, and an Env protein, and/or (ii) further comprise a lentiviral Tat protein and/or a lentiviral Rev protein.

11. The method or packaging cell of any one of claims 1-7, wherein the viral particle is an AAV particle, optionally wherein the AAV particle is an AAV1, AAV2, AAV5, AAV6, or AAV8 particle.

12. The method or packaging cell of claim 11, wherein the gene of interest is framed by a left and a right inverted terminal repeat (ITR) sequence.

13. The method or packaging cell of claim 11 or 12, wherein the one or more helper factors are selected from the group consisting of a Rep78 protein, a Rep 52 protein, an AAP protein, a capsid protein, and combination thereof, optionally wherein the one or more helper factors comprise a Rep78 protein, a Rep 52 protein, an AAP protein, and a capsid protein.

14. A system for producing a lentiviral particle, comprising
one or more constructs encoding one or more helper factors for assembling transduction-competent lentiviral particles;
an *in vitro* mRNA transcription system for transcribing mRNAs from the one or more constructs encoding one or more helper factors;
a packaging cell; and
a transfection reagent for introducing the *in vitro* transcribed mRNAs into the packaging cell.

15. A system for producing an AAV particle, comprising
one or more constructs encoding one or more helper factors for assembling transduction-competent AAV particles;
an *in vitro* mRNA transcription system for transcribing mRNAs from the one or more constructs encoding one or more helper factors;
a packaging cell; and
a transfection reagent for introducing the *in vitro* transcribed mRNAs into the packaging cell.
